# FASCICULE DE BREVET EUROPEEN

(11) **EP 1 169 316 B1**
(45) Date de publication et mention de la délivrance du brevet: **05.03.2003**
(21) Numéro de dépôt: 00918930.9
(22) Date de dépôt: 31.03.2000
(51) Int. Cl.: C07D 339/04, C07C 323/60, C07D 409/12, A61K 31/385, A61P 25/28

(54) **NOUVEAUX DERIVES DE L'ACIDE LIPOIQUE, LEUR PREPARATION ET LES COMPOSITIONS PHARMACEUTIQUES LES CONTENANT**
LIPONSAÜREDERIVATE, DEREN HERSTELLUNG UND DIESE ENTHALTENDE PHARMAZEUTISCHE ZUSAMMENSTELLUNGEN
NOVEL LIPOIC ACID DERIVATIVES, THEIR PREPARATION, AND PHARMACEUTICAL COMPOSITIONS CONTAINING THEM

(30) Priorité: 02.04.1999 FR 9904132; 24.02.2000 FR 0002315
(43) Date de publication de la demande: 09.01.2002
(73) Titulaire: SOCIETE DE CONSEILS DE RECHERCHES ET D'APPLICATIONS SCIENTIFIQUES (S.C.R.A.S.), 75016 Paris (FR)
(72) Inventeur: HARNETT, Jeremiah, F-91190 Gif-sur-Yvette (FR); AUGUET, Michel, F-91120 Palaiseau (FR); CHABRIER de LASSAUNIERE, Pierre-Etienne, F-75016 Paris (FR)
(74) Mandataire: Bourgouin, André
(86) Numéro de dépôt international: FR0000814
(87) Numéro de publication internationale: WO00059899

(56) Documents cités:
- EP-A- 0 869 126
- GB-A- 1 014 020
- CHEMICAL ABSTRACTS, vol. 107, no. 8, 1987 Columbus, Ohio, US; abstract no. 59736a, page 18; XP002129559 & JP 06 250330 A (IDEMITSU KOSAN) 9 septembre 1994 (1994-09-09)

## Description

La présente invention a pour objet des nouveaux dérivés de l'acide lipoïque, lesquels présentent une activité inhibitrice des enzymes NO-synthases produisant le monoxyde d'azote NO et sont des agents permettant la régénération d'antioxydants ou d'entités piégeuses des formes réactives de l'oxygène (ROS pour *"reactive oxygen species")* et intervenant d'une façon plus générale dans le statut rédox des groupement thiols. Ces antioxydants ou entités piégeuses des formes réactives de l'oxygène peuvent être d'origine naturelle, comme par exemple la vitamine E ou le glutathion, ou d'origine synthétique comme certains produits piégeurs de ROS ou des produits présentant à la fois une activité inhibitrice des enzymes NO-synthases et une activité piégeuse de ROS. Des exemples de tels produits d'origine synthétique peuvent notamment être trouvés dans les demandes de brevet PCT WO 96/09653, WO 98/42696 et WO 98/58934.

L'invention concerne donc en particulier les dérivés correspondant à la formule générale **(I)** définie ci-après, leurs méthodes de préparation, les préparations pharmaceutiques les contenant et leur utilisation à des fins thérapeutiques, en particulier leur utilisation en tant qu'inhibiteurs des NO-synthases et agents permettant la régénération d'antioxydants ou d'entités piégeuses des ROS et intervenant d'une façon plus générale dans le statut rédox des groupement thiols.

Compte tenu du rôle potentiel du NO, des ROS et du métabolisme du glutathion en physiopathologie, les nouveaux dérivés décrits répondant à la formule générale (I) peuvent produire des effets bénéfiques ou favorables dans le traitement de pathologies où le monoxyde d'azote et le métabolisme du glutathion ainsi que le statut rédox des groupement thiols sont impliqués, et notamment :
- troubles cardiovasculaires et cérébrovasculaires comprenant par exemple l'athérosclérose, la migraine, l'hypertension artérielle, le choc septique, les infarctus
- cardiaques ou cérébraux d'origine ischémique ou hémorragiques, les ischémies et les thromboses ;
- troubles du système nerveux central ou périphérique comme par exemple les maladies neurodégénératives où l'on peut notamment citer les infarctus cérébraux, l'hémorragie sub arachnoïde, le vieillissement, les démences séniles, y compris la maladie d'Alzheimer, la chorée de Huntington, la maladie de Parkinson, la maladie de Creutzfeld Jacob et les maladies à prions, la sclérose latérale amyotrophique mais aussi la douleur, les traumatismes cérébraux ou de la moelle épinière, l'addiction aux opiacées, à l'alcool et aux substances induisant une accoutumance, les troubles de l'érection et de la reproduction, les désordres cognitifs, les encéphalopathies, les encéphalopathies d'origine virale ou toxique, la dépression, l'anxiété, la schizophrénie, l'épilepsie, les troubles du sommeil, les troubles alimentaires (anorexie, boulimie...) ;
- troubles du muscle squelettique et des jonctions neuromusculaires (myopathie, myosite) ainsi que les maladies cutanées ;
- les maladies prolifératives et inflammatoires comme par exemple l'athérosclérose, l'hypertension pulmonaire, la détresse respiratoire, la glomérulonéphrite, la cataracte, l'hypertension portale, le psoriasis, l'arthrose et l'arthrite rhumatoïde, les fibroses, les amyloïdoses, les inflammations du système gastro-intestinal (colite, maladie de Crohn) ou du système pulmonaire et des voies aériennes (asthme, sinusites, rhinites) ainsi que les hypersensibilités de contact ou retardées ;
- les transplantations d'organes ;
- les maladies auto-immunes et virales comme par exemple le lupus, le sida, les infections parasitaires et virales, le diabète et ses complications incluant les rétinopathies, les néphropathies et les polyneuropathies, la sclérose en plaques, les myopathies ;
- le cancer ;
- les maladies génétiques autosomiques comme la maladie d'Unverricht-Lunborg ;
- les maladies neurologiques associées à des intoxications (empoisonnement au Cadmium, inhalation de n-hexane, pesticide, herbicide), à des traitements (radiothérapie) ou à des désordres d'origine génétique (maladie de Wilson);
- l'impuissance liée au diabète ;
- toutes les pathologies caractérisées par une production ou un dysfonctionnement de monoxyde d'azote et/ou du métabolisme du glutathion et du statut rédox des groupement thiols.

Dans l'ensemble de ces pathologies, il existe des évidences expérimentales démontrant l'implication du monoxyde d'azote ou d'un dysfonctionnement du métabolisme du glutathion (Kerwin et al., Nitric oxide : a new paradigm for second *messengers, J. Med. Chem.* **38**, 4343-4362, 1995 ; Packer et al., Alpha-lipoic acid as biological antioxidant, *Free Radical Biology & Medicine* **19**, 227-250, 1995). C'est le cas notamment dans la maladie de Parkinson qui illustre l'invention (Beal MF, Excitotoxicity and nitric oxide in Parkison's disease pathogenesis, *Ann. Neurol.* **44**[Suppl I], S110-S114, 1998; Donato A et al., Gluthathion in Parkinson's disease: a link between oxidative stress and mitochondrial damage, *Ann. Neurol.* **32**, S111 -S115, 1992). Dans ce contexte, les médicaments pouvant inhiber la formation du monoxyde d'azote ou rétablir la fonctionnalité biologique des groupements thiols ou du glutathion peuvent apporter des effets bénéfiques.

Par ailleurs les inventeurs ont déjà décrit dans des brevets antérieurs des inhibiteurs de NO Synthases et leur utilisation (brevets US 5,081,148; US 5,360,925), et plus récemment l'association de ces inhibiteurs avec des produits possédant des propriétés antioxydantes ou antiradicalaires (demande de brevet PCT WO 98/09653). Ils ont aussi décrit des dérivés d'amidines dans les demandes de brevet PCT WO 98/42696 et WO 98/58934 et des dérivés d'aminopyridines dans la demande de brevet PCT WO 00/02860. Ces dérivés d'amidines ou d'aminopyridines présentent la particularité d'être à la fois des inhibiteurs de NO Synthases et des inhibiteurs de ROS.

La présente invention a pour objet de nouveaux dérivés de l'acide lipoïque, leur préparation et leur application en thérapeutique.

L'invention concerne donc un produit de formule générale **(I)**, caractérisée en ce qu'elle comprend les produits de sous-formules **(I)a** et **(I)b** 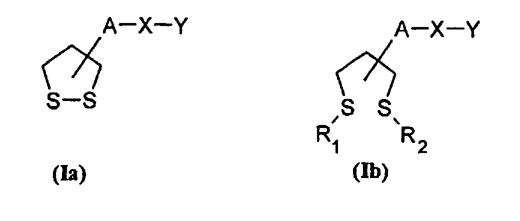 dans lesquelles
R₁ et R₂ représentent indépendamment un atome d'hydrogène ou un radical alkyle linéaire ou ramifié de 1 à 6 atomes de carbone ;
A représente l'un des radicaux -(CH₂)ₘ-NR₃-CO(CH₂)ₙ-, -(CH₂)ₘ-CO-NR₃-(CH₂)ₙ-, -(CH₂)ₘ-NR₃-(CH₂)ₙ-, -(CH₂)ₘ-CO-NR₃-(CH₂)ₚ-NR₄-(CH₂)ₙ-, -(CH₂)ₘ-NR₃-CO-NR₄-(CH₂)ₙ- ou -(CH₂)ₘ-,
m et n étant des entiers de 0 à 6,
p étant un entier de 2 à 6,
et R₃ et R₄ représentant indépendamment un atome d'hydrogène ou un radical alkyle linéaire ou ramifié de 1 à 6 atomes de carbone ;
X représente un radical 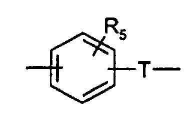 dans lequel le groupe T, qui est attaché au groupe Y, représente un radical -(CH₂)ᵢ- dans lequel i représente un entier compris entre 0 et 6, et R₅ représente un atome d'hydrogène, un radical alkyle linéaire ou ramifié ayant de 1 à 6 atomes de carbone ou un radical -(CH₂)ₘ-Q dans lequel Q représente un atome halogène ou un radical hydroxy, cyano, amino, alkoxy, alkylthio, alkylamino ou dialkylamino, ou encore R₅ représente un hétérocycle de 5 à 6 chaînons dont les chaînons hétérocycliques sont choisis parmi les radicaux -O-, -N(R₆)- et -S-, R₆ représentant un atome d'hydrogène, un alkyle linéaire ou ramifié ayant de 1 à 6 atomes de carbone ou la liaison vers le cycle phényle du radical X ;
ou encore X représente un radical -(CH₂)_{q}- dans lequel q représente un entier de 0 à 6 ;
et enfin Y représente l'un des radicaux 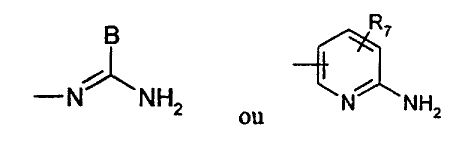 dans lesquels :
B représente un radical alkyle linéaire ou ramifié ayant de 1 à 6 atomes de carbone, aryle carbocyclique ou hétérocyclique à 5 ou 6 chaînons contenant de 1 à 4 hétéroatomes choisis parmi O, S, N et notamment les radicaux thiophène, furanne, pyrrole ou thiazole, le radical aryle étant éventuellement substitué par un ou plusieurs groupes choisis parmi les radicaux alkyle, alkényle ou alkoxy linéaires ou ramifiés ayant de 1 à 6 atomes de carbone,
ou B représente NR₈R₉, dans lequel R₈ et R₉ représentent, indépendamment, un atome d'hydrogène ou un radical alkyle linéaire ou ramifié ayant de 1 à 6 atomes de carbone,
ou l'un de R₈ et R₉ représente un radical nitro tandis que l'autre représente un atome d'hydrogène ou un radical alkyle linéaire ou ramifié ayant de 1 à 6 atomes de carbone.
ou encore R₈ et R₉ pris ensemble forment avec l'atome d'azote un hétérocycle non aromatique de cinq à six chaînons, les éléments de la chaîne étant choisis parmi un groupe composé de -CH₂-, -NH-, -O- ou -S-,
ou encore B représente un radical SR₁₀ dans lequel R₁₀ représente un atome d'hydrogène ou un radical alkyle linéaire ou ramifié ayant de 1 à 6 atomes de carbone.
et R₇ représente un atome d'hydrogène ou un radical alkyle linéaire ou ramifié de 1 à 6 atomes de carbone ;
ou un sel d'un produit de formule générale **(I)**.

De préférence, lorsque R₅ représente un hétérocycle de 5 à 6 chaînons, R₅ sera l'un des hétérocycles suivants : pyrrole, imidazole, pyrazole, triazole, thiazolidine, pyrrolidine, pipéridine, pipérazine, N-alkyl-pipérazine, thiomorpholine, morpholine, azétidine.

Par ailleurs, l'invention concerne en particulier les produits de formule générale **(I)** définie précédemment, dans lesquels se retrouvent, indépendamment, les caractéristiques suivantes :
- A représente l'un des radicaux -(CH₂)ₘ-NR₃-CO-(CH₂)ₙ-, -(CH₂)ₘ-CO-NR₃-(CH₂)ₙ-, ou -(CH₂)ₘ-NR₃-CO-NR₄-(CH₂)ₙ, m représentant un entier de 0 à 4 et n un entier de 0 à 6, et R₃ et R₄ représentant indépendamment un atome d'hydrogène ou un radical alkyle linéaire ou ramifié de 1 à 6 atomes de carbone ;
- X représente le radical 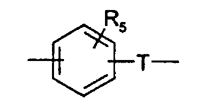 dans lequel le groupe T, qui est attaché au groupe Y, représente un radical -(CH₂)ᵢ- dans lequel i représente 0 ou 1, et R₅ représente un atome d'hydrogène, un radical alkyle linéaire ou ramifié ayant de 1 à 6 atomes de carbone ou un radical -(CH₂)ₘ-Q dans lequel Q représente un atome halogène ou un radical hydroxy, cyano, amino, alkoxy, alkylamino ou dialkylamino, ou encore R₅ représente un hétérocycle de 5 à 6 chaînons dont les chaînons hétérocycliques sont choisis parmi les radicaux -O-, -N(R()- et -S-, R₆ représentant un atome d'hydrogène, un alkyle linéaire ou ramifié ayant de 1 à 6 atomes de carbone ou la liaison vers le cycle phényle du radical X ; ou
- Y représente le radical 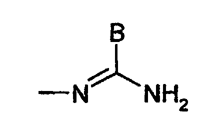
dans lequel B représente un radical alkyle linéaire ou ramifié ayant de 1 à 6 atomes de carbone, aryle carbocyclique ou hétérocyclique à 5 ou 6 chaînons contenant de 1 à 4 hétéroatomes choisis parmi O, S, N et notamment les radicaux thiophène. furanne, pyrrole ou thiazole, le radical aryle étant éventuellement substitué par un ou plusieurs groupes choisis parmi les radicaux alkyle, alkényle ou alkoxy linéaires ou ramifiés ayant de 1 à 6 atomes de carbone.

Plus particulièrement, l'invention concerne les produits suivants décrits dans les exemples (parfois sous forme de sels) :
- N-{4-[[(2-thiényl)(imino)méthyl]amino]phényl}-1,2-dithiolane-3-pentanamide ;
- N-{2-{4[[(2-thiényl)(imino)méthyl]amino]phényl}éthyl}-1,2-dithiolane-3-pentanamide ;
- N-{2-{4[[(2-thiényl)(imino)méthyl]amino]phényl}éthyl}-1,2-dithiolane-3-acétamide ;
- N-[4-(6-amino-4-méthyl-2-pyridinyl)butyl]-1,2-dithiolane-3-pentanamide ;
- N-[4-(6-amino-4-méthyl-2-pyridinyl)butyl]-1,2-dithiolane-3-acétamide ;
- N-(4-{[amino(2-thiényl)méthylidène]amino}phényl)-2(1,2-dithiolan-3-yl)acétamide;
- N-(4-{[amino(2-thiényl)méthylidènejamino}benzyl)-5-(1,2-dithiolan-3-yl)pentanamide ;
- N-(5-{[amino(2-thiényl)méthylidène]amino}-2-méthoxyphényl)-5-(1,2-dithiolan-3-yl)pentanamide ;
- N-[5-{[amino(2-thiényl)méthylidène]amino}-2-(diméthylamino)benzyl]-5-(1,2-dithiolan-3-yl)pentanamide ;
- N-[5-{[amino(2-thiényl)méthylidène]amino}-2-(1H-pyrrol-1-yl)benzyl]-5-(1,2-dithiolan-3-yl)pentanamide ;
et les sels de ces composés.

L'invention offre de plus un certain nombre de procédés pour accéder aux produits de formule générale **(I)** décrits ci-dessus, procédés dont les conditions préférées sont décrites plus loin.

L'invention concerne donc en particulier un procédé de préparation d'une amidine de formule générale **(I)** tel que définie précédemment, caractérisé en ce que l'on fait réagir l'intermédiaire de formule générale **(II)**. 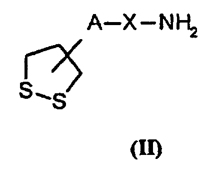 dans laquelle A et X sont tels que définis ci-dessus,
avec l'intermédiaire de formule générale **(I.i)** 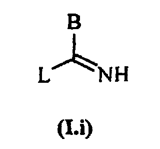 dans laquelle B est tel que défini ci-dessus et L représente un groupe partant, par exemple un radical alkoxy, alkylthio, acide sulfonique, halogénure, alcool arylique ou tosyle.

L'invention concerne de plus un procédé de préparation d'un composé de formule générale **(I)** dans laquelle A représente un radical -(CH₂)ₘ-CO-NR₃-(CH₂)ₙ- tel que défini précédemment, caractérisé en ce que l'on fait réagir l'intermédiaire de formule générale **(VII)** a représentée ci-dessous

HN(R₃)-A'-X-Y **(VII)a**

R₃, X et Y étant tels que définis ci-dessus et A' représentant le radical -(CH₂)ₙ-, n étant tel que défini ci-dessus, avec le composé de formule générale **(I.vi)** 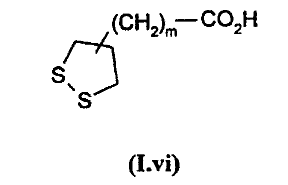 m étant tel que défini ci-dessus.

L'invention concerne de plus un procédé de préparation d'un composé de formule générale **(I)** dans laquelle A représente un radical -(CH₂)ₘ-NR₃-CO-NR₄-(CH₂)ₙ tel que défini précédemment, caractérisé en ce que l'on fait réagir l'intermédiaire de formule générale **(VII)b** représentée ci-dessous

HN(R₄)-A'-X-Y **(VII)b**

R₄, X et Y étant tels que définis ci-dessus et A' représentant le radical -(CR₂)ₙ-, n étant tel que défini ci-dessus, avec le composé de formule générale **(I.vi)** 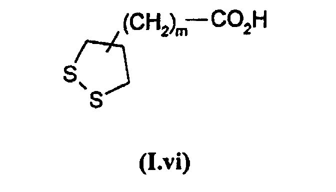 m étant tel que défini ci-dessus, et avec du diphénylphosphorylazide en présence d'une base comme par exemple la triéthylamine.

L'invention concerne également un procédé de préparation d'un composé de formule générale **(I)** dans laquelle B est une amine, caractérisé en ce que l'on fait réagir l'intermédiaire de formule générale **(II)** 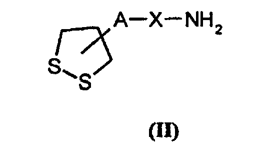 dans laquelle A et X sont tels que définis ci-dessus,
a) avec l'intermédiaire de formule générale **(I.ii)** 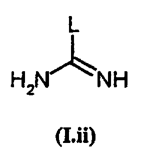 dans laquelle L représente un groupe partant, par exemple un radical alkoxy, alkylthio, acide sulfonique, halogénure, alcool arylique ou tosyle,
b) ou avec l'intermédiaire de formule générale **(I.iii)** 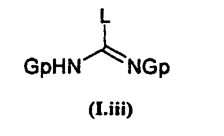 dans laquelle L représente un groupe partant, par exemple un radical alkoxy, alkylthio. acide sulfonique, halogénure, alcool arylique ou tosyle, et Gp un groupe protecteur de type carbamate, par exemple le groupe t-butoxycarbonyle,
   cette réaction étant suivie, dans le cas où l'on opte pour la réaction avec le composé de formule générale **(Liii)**, par une hydrolyse en présence d'un acide fort, par exemple l'acide trifluoroacétique,
c) ou avec le dérivé de formule **(I.iv)** (N-méthyl-N'-nitro-N-nitrosoguanidine) 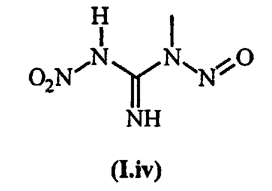
d) ou enfin avec le dérivé de formule **(I.v)** dans laquelle Gp représente un groupe protecteur

   S=· =N-Gp. **(I.v)**

Dans certains cas, les composés selon la présente invention peuvent comporter des atomes de carbone asymétriques, et donc posséder deux formes énantiomères possibles, c'est-à-dire les configurations "R" et "S". La présente invention inclut les deux formes énantiomères et toutes combinaisons de ces formes, y compris les mélanges racémiques "RS". Dans un souci de simplicité, lorsqu'aucune configuration spécifique n'est indiquée dans les formules de structure, il faut comprendre que les deux formes énantiomères et leurs mélanges sont représentés.

Par ailleurs, dans la présente demande, lorsqu'il n'est pas donné plus de précision, on entend par alkyle un radical alkyle linéaire ou ramifié comptant de 1 à 6 atomes de carbone. Par alkényle, lorsqu'il n'est pas donné plus de précision, on entend un radical alkyle linéaire ou ramifié comptant de 1 à 6 atomes de carbone et présentant au moins une insaturation (double liaison).

Par radicaux alkylthio, alkoxy, alkylamino, dialkylamino et alkényle, on entend respectivement les radicaux alkylthio, alkoxy, alkylamino, dialkylamino et alkényle dont le radical alkyle a la signification indiquée précédemment.

Par alkyle linéaire ou ramifié ayant de 1 à 6 atomes de carbone, on entend en particulier les radicaux méthyle, éthyle, propyle, isopropyle, butyle, isobutyle, sec-butyle et tert-butyle, pentyle, néopentyle, isopentyle, hexyle, isohexyle. Par halogène, on entend les atomes de fluor, de chlore, de brome ou d'iode.

L'invention a également pour objet, à titre de médicaments, les composés décrits précédemment ou leurs sels pharmaceutiquement acceptables. Elle concerne aussi des compositions pharmaceutiques contenant ces composés ou leurs sels pharmaceutiquement acceptables, et l'utilisation de ces composés ou de leurs sels pharmaceutiquement acceptables pour fabriquer des médicaments destinés à assurer la double fonction d'inhibition de la NO synthase et de régénération d'anti-oxydants.

Par sel pharmaceutiquement acceptable, on entend notamment des sels d'addition d'acides inorganiques tels que chlorhydrate, bromhydrate, iodhydrate, sulfate, phosphate, diphosphate et nitrate ou d'acides organiques tels que acétate, maléate, fumarate, tartrate, succinate, citrate, lactate, méthanesulfonate, p-toluènesulfonate, pamoate, oxalate et stéarate. Entrent également dans le champ de la présente invention, lorsqu'ils sont utilisables, les sels formés à partir de bases telles que l'hydroxyde de sodium ou de potassium. Pour d'autres exemples de sels pharmaceutiquement acceptables, on peut se référer à "Pharmaceutical salts", *J. Pharm. Sci.* **66**:1 (1977).

L'invention a aussi pour objet l'utilisation d'un produit de formule générale **(I)** ou d'un sel pharmaceutiquement acceptable de ce produit pour fabriquer un médicament destiné à traiter des pathologies dans lesquelles le monoxyde d'azote et le statut rédox des groupements thiols sont impliqués, pathologies telles que les troubles du système nerveux central ou périphérique particulièrement bien représentés par la maladie de Parkinson, les troubles cérébrovasculaires, les maladies prolifératives et inflammatoires, les vomissements, le choc septique, les pathologies résultant des irradiations radioactives, des radiations solaires ou des transplantations d'organes, les maladies autoimmunes et autosomales, les cancers et toutes les pathologies caractérisées par une production ou un dysfonctionnement impliquant le monoxyde d'azote et impliquant le statut rédox des groupements thiols.

L'invention a encore pour objet l'utilisation d'un produit de formule générale **(I)** ou d'un sel pharmaceutiquement acceptable de ce produit, pour fabriquer un médicament destiné à traiter des troubles cérébrovasculaires tels que la migraine, les infarctus cérébraux d'origine ischémique ou hémorragique, les ischémies et les thromboses.

L'invention a enfin pour objet l'utilisation d'un produit de formule générale **(I)** ou d'un sel pharmaceutiquement acceptable de ce produit pour fabriquer un médicament destiné à traiter les troubles du système nerveux central ou périphériques tels que les maladies neurodégénératives, la douleur, les traumatismes cérébraux ou de la moëlle épinière, l'addiction aux drogues opiacées, à l'alcool et aux substances induisant une accoutumance, les troubles de l'érection et de la reproduction, les désordres cognitifs, les encéphalopathies, la dépression, l'anxiété, la schizophrénie, l'épilepsie, les troubles du sommeil et les troubles alimentaires.

Les compositions pharmaceutiques peuvent être sous forme d'un solide, par exemple des poudres, des granules, des comprimés, des gélules, des liposomes ou des suppositoires. Les supports solides appropriés peuvent être, par exemple, le phosphate de calcium, le stéarate de magnésium, le talc, les sucres, le lactose, la dextrine, l'amidon, la gélatine, la cellulose, la cellulose de méthyle, la cellulose carboxyméthyle de sodium, la polyvinylpyrrolidine et la cire.

Les compositions pharmaceutiques contenant un composé de l'invention peuvent aussi se présenter sous forme liquide, par exemple, des solutions, des émulsions, des suspensions ou des sirops. Les supports liquides appropriés peuvent être, par exemple, l'eau, les solvants organiques tels que le glycérol ou les glycols, de même que leurs mélanges, dans des proportions variées, dans l'eau.

L'administration d'un médicament selon l'invention pourra se faire par voie topique, orale, parentérale, par injection intramusculaire, etc.

La dose d'administration envisagée pour médicament selon l'invention est comprise entre 0,1 mg à 10 g suivant le type de composé actif utilisé.

Conformément à l'invention, on peut préparer les composés de formule générale **(I)** par les procédés ci-après.

### PRÉPARATION DES COMPOSÉS DE FORMULE GÉNÉRALE (I)

### A) Préparation des composés de formule générale (I) où Y représente

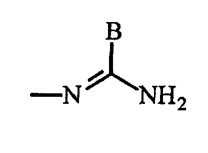

### PREMIÈRE APPROCHE :

Les composés de formule générale **(I)** peuvent être préparés à partir des intermédiaires de formule générale **(II)**, **(III)** et **(IV)** selon le schéma 1 où A, B, X et Y, sont tels que définis ci-dessus et Gp est un groupe protecteur de type carbamate.

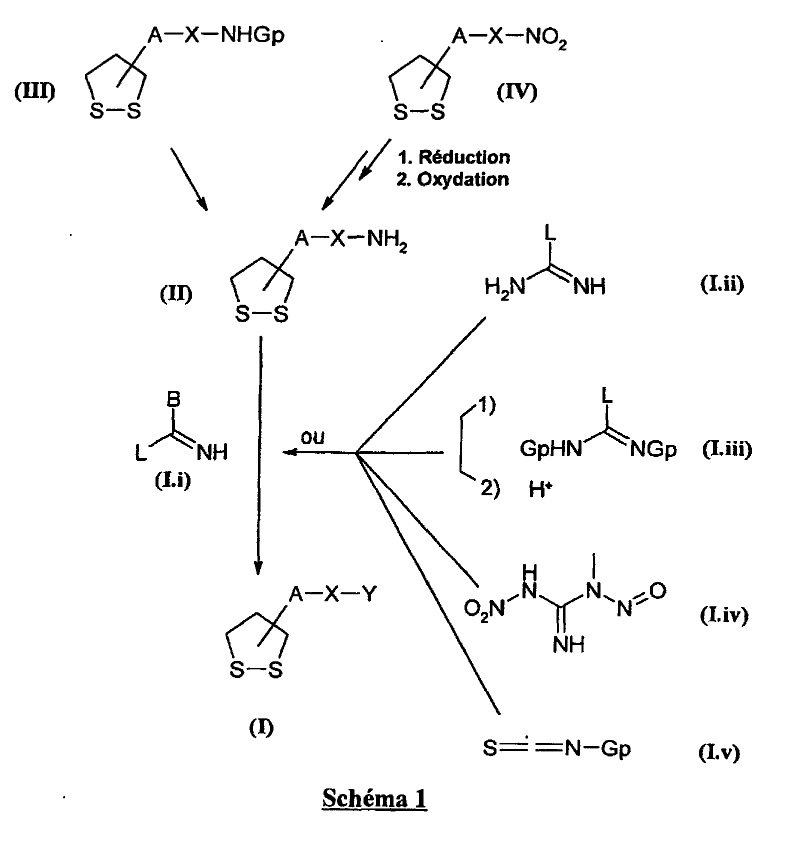

Les dérivés d'aniline et d'amines de formule générale **(II)**, peuvent être condensés sur des composés de formule générale **(I.i)**, dans lesquels L représente un groupe partant (en particulier un radical alkoxy, alkylthio, acide sulfonique, halogénure, alcool arylique ou tosyle), pour conduire aux composés finaux de formule générale **(I)** de type amidine substitué (cf. schéma 1). Par exemple, pour B = thiophène, on peut condenser les dérivés de formule générale **(II)** sur l'iodhydrate de S-méthylthiophène thiocarboxamide, préparé selon une méthode de la littérature *(Ann. Chim.* (1962), **7**, 303-337). La condensation peut s'effectuer par chauffage dans un alcool (par exemple dans du méthanol ou de l'isopropanol), éventuellement en présence de DMF à une température de préférence comprise entre 50 et 100 °C pour une durée généralement comprise entre quelques heures et une nuit.

Dans les cas où B = SR₁₀, par exemple S-CH₃, ceux-ci peuvent être préparés par la condensation des aminés ou anilines de formule générale **(II)** avec l'isothiocyanate **(I.v)** dans laquelle Gp représente un groupe protecteur tel que par exemple le groupe benzoyle. On déprotége ensuite par clivage du groupe protecteur dans des conditions convenables et la thiourée formée est finalement traitée avec, par exemple, un halogénoalcane pour conduire aux composés finaux de formule générale **(I)**.

Dans les cas où B = NR₈R₉, les composés finaux de formule générale **(I)** sont des guanidines. Celles-ci peuvent être préparées, par exemple, par la condensation des aminés ou anilines de formule générale **(II)** avec les dérivés de formule générale **(I.ii)** ou **(I,iii).** Les réactifs de formule générale **(I.ii)** dans lesquels L représente, par exemple, un cycle pyrazole sont condensés sur les amines de formule générale **(II)** selon les conditions décrites dans la littérature (*J. Org. Chem.* (1992) **57**, 2497-2502) de même pour les réactifs de formule générale **(I.iii)** dans lesquels L représente, par exemple, un cycle pyrazole et Gp le groupement tBuOCO *(Tetrahedron Lett.* (1993) **34** (21), 3389-3392) ou bien lorsque L représente le groupement -N-SO₂-CF₃ et Gp le groupement tBuOCO (*J Org. Chem.* (1998) **63**, 3804-3805). Lors de l'ultime étape de la synthèse, la déprotection de la fonction guanidine est effectuée en présence d'un acide fort tel que par exemple l'acide trifluoroacétique.

Dans les cas où B = -NHNO₂ les composés finaux de formule générale **(I)** peuvent être préparés, par exemple, par la condensation des amines ou anilines de formule générale **(II)** avec le réactif de formule **(I.iv)** (N-méthyl-N'-nitro-N-nitrosoguanidine) selon les conditions décrites dans la littérature.(J. Amer. Chem. Soc. (1947), **69**, 3028-3030).

Les composés de formule générale **(I)b**, sont obtenus à partir des composés de formule générale **(I)a** où A, X et Y sont tels que définis ci-dessus. La transformation des composés lipoïques de formule générale **(I)a** en dérivés dihydrolipoïques de formule générale **(I)b** dans laquelle R₁ = R₂ = H est effectué dans un solvant alcoolique tel que, par exemple, le méthanol en présence d'un agent réducteur tel que par exemple NaBH_{4,} NaBH₃CN ou LiAlH₄. Les composés de formule générale **(I)b** pour lesquels R₁ et R₂ ne sont pas H sont préparés en faisant réagir les composés de formule générale **(I)b** dans lesquels R₁ = R₂ = H avec un composé de formule R₁-Hal et / ou R₂-Hal (Hal = atome halogène) où R₁ et R₂ sont tels que définis ci-dessus et l'atome halogène est un groupe partant. La réaction est effectuée, par exemple, dans un solvant approprié tel que le THF, l'acétone, l'acétate d'éthyle en présence d'une base telle que K₂CO₃ ou la triéthylamine, pour conduire aux intermédiaires de formule générale **(I)b**.

### Préparation des composés de formule générale (II) :

Les intermédiaires de formule générale **(II)**, sont obtenus à partir de la coupure d'un groupe protecteur (Gp) ou par réduction d'un groupement nitro.

Les intermédiaires de formule générale **(II)**, dans lesquels A et X sont tels que définis ci-dessus, peuvent être préparés à partir des intermédiaires de formule générale **(III)** ou **(IV)**, schéma 1, qui sont des composés comportant respectivement une amine ou aniline protégée (NHGp) sous forme, par exemple, d'un carbamate ou un groupement nitro. Dans le cas particulier des groupes BOC, ceux-ci sont déprotégés classiquement à l'aide de TFA ou HCl pour conduire finalement aux amines et anilines primaires de formule générale **(II)**. La réduction de la fonction nitro des intermédiaires de formule générale **(IV)**, schéma 1, dans lesquels A, et X sont tels que définis ci-dessus, est effectuée, par exemple, en chauffant le produit dans un solvant approprié tel que l'acétate d'éthyle avec un peu d'éthanol en présence de SnCl₂ (*J*. *Heterocyclic Chem.* (1987), 24, 927-930 *; Tetrahedron Letters* (1984), 25 (8), 839-842) en présence de SnCl₂ / Zn (*Synthesis.* (1996), **9**,1076-1078), à l'aide de NaBH₄-BiCl₃ (*Synth. Com.* (1995) 25 (23), 3799-3803) dans un solvant tel que l'éthanol, ou alors en utilisant du Ni Raney additionné d'hydrate d'hydrazine (*Monatshefte für Chemie,* (1995), 126, 725-732), ou bien à l'aide d'indium dans un mélange d'éthanol et de chlorure d'ammonium à reflux (*Synlett* (1998) **9**, 1028). Ensuite, le produit de double réduction est ré-oxydé en présence de chlorure ferrique (FeCl₃) (*Synlett (1991)* **10**, *717-718* ) ou d'iode (*Tetrahedron Letters. (1997),* **38** (33), 5785-5788 ) pour conduire finalement aux amines et anilines contenant à nouveau le dithiolane de formule générale **(II)**.

### Préparation des composés de formule générale (III) et (IV) :

### Synthèse des carboxamides de formule générale (III) et (IV) :

Les carboxamides de formule générale **(III)** et **(IV)**, schéma 2, dans lesquels A, X, R₃ et m sont tels que définis ci-dessus, sont préparés par condensation des acides de formule générale **(I.vi)** avec les amines ou anilines mono-protégées de formule générale **(V)** ou les dérivés nitro de formule générale **(VI)** dans lesquels A' représente le radical -(CH₂)ₙ-. Le radical Rₓ dans les schémas de synthèse de la présente demande, signifie, selon le cas, R₃ ou R₄. Les liaisons carboxamides sont formées dans des conditions classiques de synthèse peptidique (M. Bodanszky et A. Bodanszky, *The Practice of Peptide Synthesis,* 145 (Springer-Verlag, 1984)) dans le THF, le dichlorométhane ou le DMF en présence d'un réactif de couplage tel que le dicyclohexylcarbodiimide (DCC), le 1,1'-carbonyldiimidazole (CDI) (*J. Med. Chem.* (1992), **35** (23), 4464-4472) ou le chlorhydrate de 1-(3-diméthylaminopropyl)-3-éthylcarbodiimide (EDC ou WSCI) (John Jones, *The chemical synthesis of peptides,* 54 (Clarendon Press, Oxford, 1991))_ou en présence de chloroformiate d'isobutyle et de N-méthylmorpholine (*Org. Prep. Proced*. *Int.,* (1975), **35**, 215). Les synthèses des acides carboxyliques de formule générale **(I.vi)** et des amines / anilines de formule générale **(V)** et **(VI)**, non commerciaux, sont décrites plus loin.

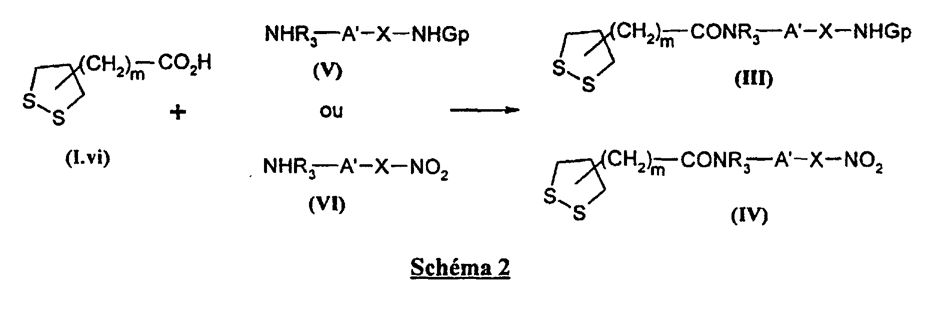

### DEUXIEME APPROCHE :

Les composés de formule générale **(I)** peuvent aussi être préparés à partir des intermédiaires de formule générale **(VII)**, **(VIIII)**, **(IX)** et **(X)** selon le schéma 3 dans lequel A, B, X et Y sont tels que définis ci-dessus, A' représente le radical -(CH₂)ₙ-, le radical Rₓ signifie, selon le cas, R₃ ou R₄, et Gp est un groupe protecteur, par exemple un groupe protecteur de type carbamate.

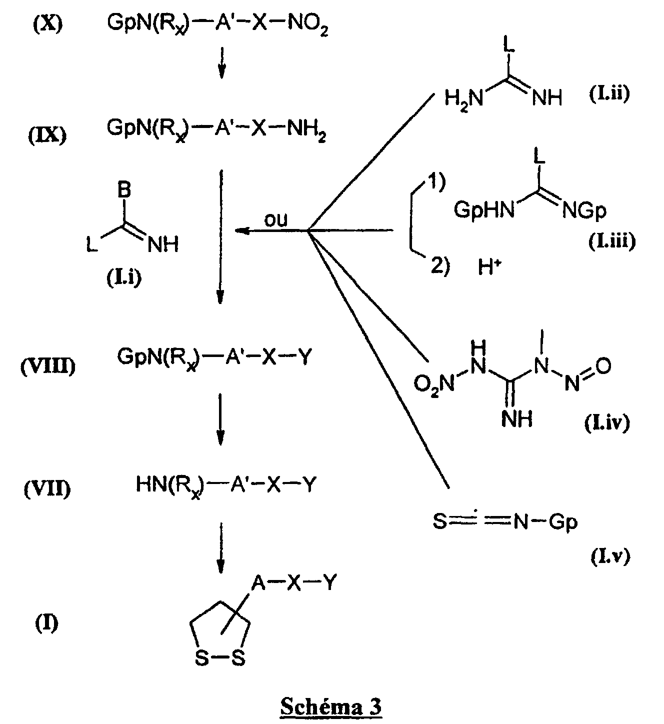

### Synthèse des carboxamides de formule générale (I):

Les carboxamides de formule générale **(I)**, schéma 4, dans lesquels A', X, R₃, Y et m sont tels que définis ci-dessus, sont préparés par condensation des acides de formule générale **(I.vi)** avec les amines / anilines de formule générale **(VII)**. Les liaisons carboxamides sont formées dans des conditions classiques de synthèse peptidique (M. Bodanszky et A. Bodanszky, *The Practice of Peptide Synthesis,* 145 (Springer-Verlag, 1984)) dans le THF, le dichlorométhane ou le DMF en présence d'un réactif de couplage tel que le dicyclohexylcarbodiimide (DCC), le 1,1'-carbonyldiimidazole (CDI) (J. *Med. Chem.* (1992), 35 (23), 4464-4472) ou le chlorhydrate de 1-(3-diméthylaminopropyl)-3-éthylcarbodiimide (EDC ou WSCI) (John Jones, *The chemical synthesis of peptides,* 54 (Clarendon Press, Oxford, 1991)) ou en présence de chloroformiate d'isobutyle et de N-méthylmorpholine (*Org. Prep. Proced. Int.,* (1975), **35**, 215). Les synthèses des acides carboxyliques de formule générale **(I.vi)** non commerciaux sont décrites plus loin.

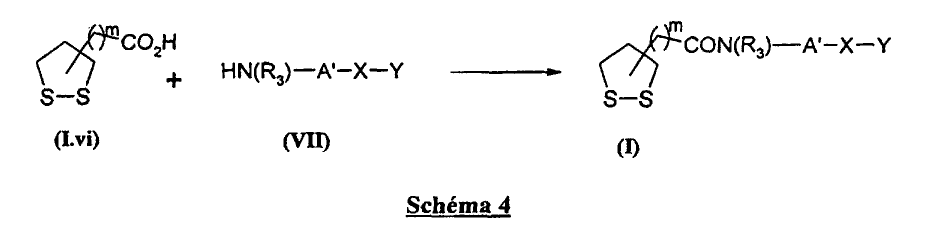

### Synthèse des urées de formule générale (I) :

Les urées de formule générale **(I)**, schéma 5, dans lesquelles m, A', R₄, X et Y sont tels que définis ci-dessus, sont préparés par condensation des acides de formule générale **(I.vi)** avec les aminés / anilines de formule générale **(VII)** dans un solvant comme le toluène en présence de diphénylphosphorylazide (DPPA) et d'une base comme par exemple la triéthylamine, de préférence pendant 2 à 3 heures et en chauffant, de préférence à une température de 40 à 110 °C, par exemple à une température de 80 °C.

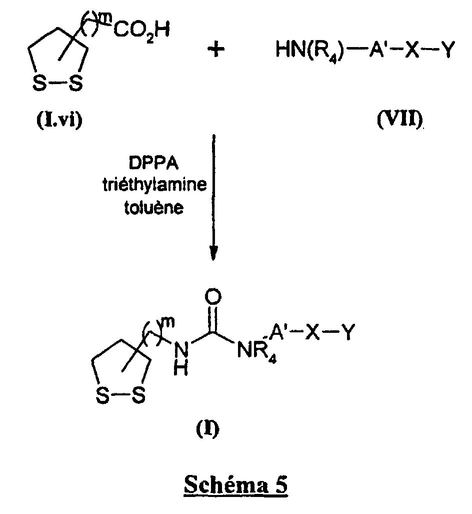

### Préparation des composés de formule générale (VII), (VIII), (IX) et (X) :

Les composés de formule générale **(VII)**, sont obtenus à partir de la coupure d'un groupe protecteur. Les composés de formule générale **(VII)**, dans lesquels Rₓ, A', X et Y sont tels que définis ci-dessus, peuvent être préparés à partir des composés de formule générale **(VIII)**, schéma 3, qui sont des composés comportant une amine protégée (NGp) sous forme, par exemple, d'un carbamate. Dans le cas particulier des groupes BOC, ceux-ci sont déprotégés classiquement à l'aide d'acide trifluoroacétique (TFA) ou de HCl , pour conduire finalement aux amines de formule générale **(VII)**.

Les composés de formule générale **(VIII)** peuvent être préparés à partir des intermédiaires de formule générale **(IX)** et **(X)** selon le schéma 3 où B, A', X, Y et Rₓ sont tels que définis ci-dessus et Gp est un groupe protecteur, par exemple de type carbamate.

Les dérivés d'aniline / d'amines de formule générale **(IX)** peuvent être condensés sur des composés de formule générale **(I.i)**, **(I.ii)**, et **(I.iii)**, dans lesquels L représente un groupe partant, ou les composés de formule générale **(I.iv)** et **(I.v)**, comme précédemment décrit pour les composés de formule générale **(I)** dans le schéma 1, pour conduire finalement aux composés de formule générale **(VIII)**, schéma 3. Dans les cas où R₃ représente le radical 2-hydroxy-4,6-diméthoxybenzyle et Gp représente t-butoxycarbonyle (BOC), ces conditions provoquent la N-débenzylation *in situ*, pour conduire directement aux composés de formule générale **(VIII)**, schéma 6.

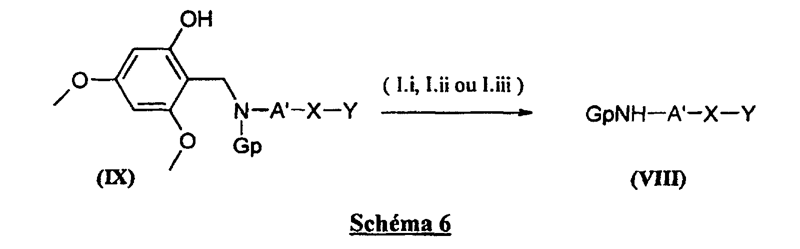

Les composés de formule générale **(IX)**, sont obtenus à partir de la réduction d'un groupement nitro des composés de formule générale **(X)**. La réduction de la fonction nitro des composés de formule générale **(X)**, schéma 3, dans lesquels Rₓ, A' et X sont tels que définis ci-dessus, est effectuée, par exemple, en chauffant le produit dans un solvant approprié tel que l'acétate d'éthyle avec un peu d'éthanol en présence de SnCl₂ (*J*. *Heterocyclic Chem.* (1987), 24, 927-930 ; *Tetrahedron Letters* (1984), 25 (8), 839-842) en présence de SnCl₂ / Zn (*Synthesis.* (1996),9,1076-1078) ou bien à l'aide de NaBH₄-BiCl₃ (*Synth. Com.* (1995) 25 (23), 3799-3803) dans un solvant tel que l'éthanol, ou alors en utilisant du Ni Raney additionné d'hydrate d'hydrazine (*Manatshefte für Chemie,* (1995), 126, 725-732), ou encore à l'aide d'indium dans un mélange d'éthanol et de chlorure d'ammonium à reflux (*Synlett (1998) 9,* 1028), pour conduire finalement aux amines et anilines primaires de formule générale **(IX)**.

La préparation des composés de formule générale **(X)** non commerciaux est décrite plus loin.

### B) Préparation des composés de formule générale (I) où Y représente :

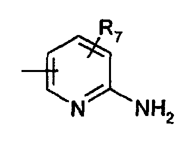

Les composés de formule générale **(I)** dans lesquels A, X, Y et R₇ sont tels que définis ci-dessus, peuvent être préparés à partir des intermédiaires de formule générale **(II)** selon la procédure illustrée dans le schéma 7.

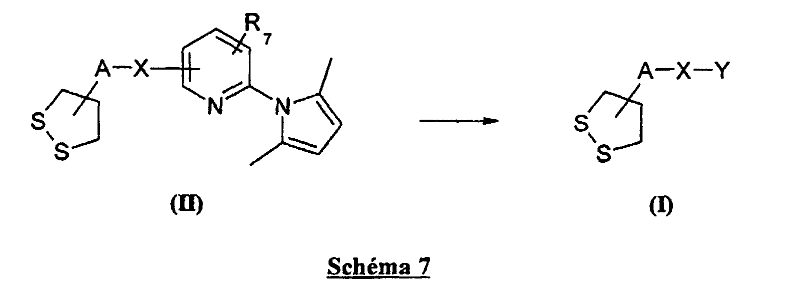

Les molécules finales de formule générale **(I)** sont obtenues après coupure du groupe protecteur 2,5-diméthylpyrrole des composés de formule générale **(II)** par chauffage en présence de chlorhydrate d'hydroxylamine, à une température variant de 60 °C à 100 °C, dans un solvant tel que par exemple l'éthanol selon un protocole expérimental décrit dans J *Chem. Soc. Perkin Trans.*(1984),**12**, 2801-2807.

### Préparation des composés de formule générale (II):

Les composés de formule générale **(II)** peuvent être préparés selon les schémas synthétiques suivants :

### 1) Méthodes d'accès aux 2-(2,5-diméthylpyrrol-1-yl)pyridine substitués de formule générale (II.x) :

**1.1)** Les précurseurs synthétiques qui conduisent aux intermédiaires de formule générale **(II)** sont préparés, schéma 8, à partir des composés de formule générale **(II.1)**, tels que par exemple la 2-(2,5-diméthylpyrrol-1-yl)-4,6-diméthylpyridine.
   Celle-ci est obtenue à partir de la 6-amino-2,4-lutidine commerciale selon un protocole expérimental décrit dans *J. Chem. Soc. Perkin Trans.,* (1984), **12**, 2801-2807. Le traitement des composés de formule générale **(II.1)** par une base forte telle que, par exemple, n-BuLi, à une température variant de -50 °C à -30 °C dans un solvant anhydre tel que l'éther éthylique, sous atmosphère inerte et en présence éventuellement de N,N,N',N'-tétraméthyléthylènediamine permet de former le dérivé lithié (intermédiaire **(II.2)**) qui en présence d'un électrophile E⁺ conduit aux adduits de formule générale **(II.x)**. 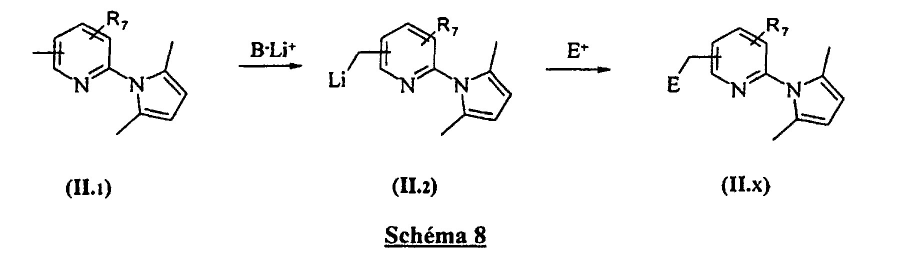
**1.2)** Parmi les électrophiles E⁺ qui peuvent réagir sur l'espèce lithiée de formule générale **(II.2)**, se trouvent par exemple des halogéno-amines protégées. Les amines de formule générale **(II.3)** sont préparées à partir de l'intermédiaire **(II.2)** qui est condensé, par exemple, sur des halogéno-amines protégées (par exemple sous forme de dérivés silylés ou de phtalimides) dans des conditions précédemment décrites. Les amines de formule générale **(II.4)** sont finalement obtenues après déprotection dans des conditions décrites dans la littérature (T.W. Greene et P.G.M. Wuts, Protective Groups in Organic Synthesis, Second édition (Wiley-Interscience, 1991)).

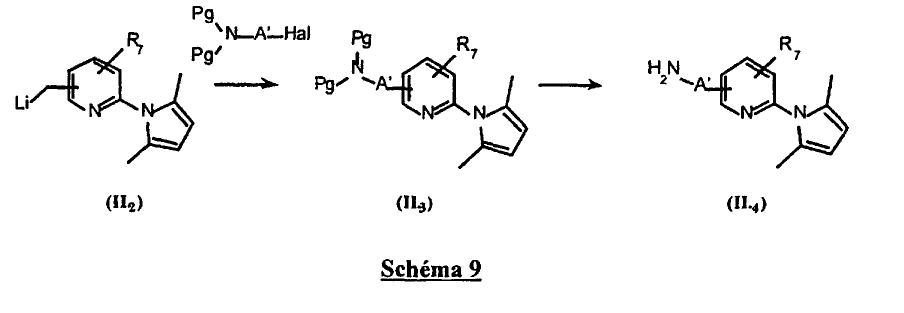

### 2) Méthodes d'accès aux composés de formule générale (II) :

### Préparation des carboxamides de formule générale (II):

Les carboxamides de formule générale **(II)**, dans lesquels m, R₃, A' et R₇ sont tels que définis ci-dessus, peuvent également être préparés, schéma 10, par condensation des acides carboxyliques de formule générale **(I.vi)** avec les amines de formule générale **(II.4)** dans les conditions précédemment décrites. La synthèse des acides carboxyliques de formule générale **(I.vi)**, non commerciaux, est décrite ci-après.

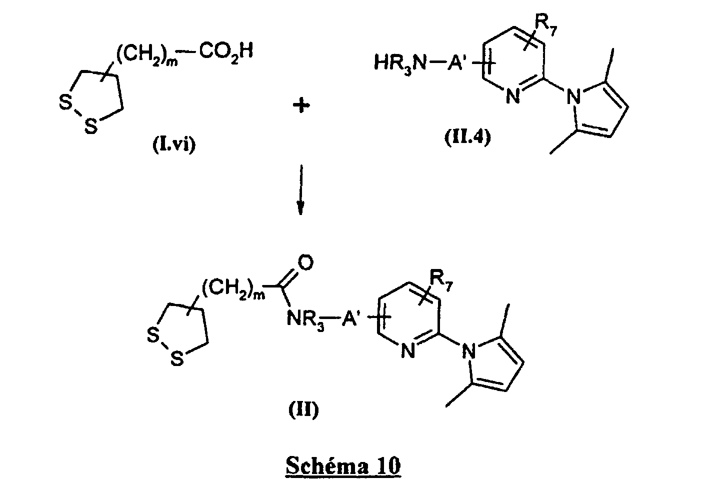

### 3) Préparation de certains intermédiaires de synthèse non commerciaux :

Les acides de formule générale **(I.vi)** non commerciaux, dans lesquels m est tel que défini ci-dessus sont accessibles à partir de méthodes de la littérature. Par exemple, l'acide trisnorlipoïque est obtenu en 5 étapes selon un protocole expérimental décrit dans *Tetrahedron Letters* (1997), **38** (33), 5785-5788.

Lorsque X représente un radical -(CH₂)_{q}-, les amines primaires sélectivement mono-protégées de formule générale **(V)** et **(IX)** dans lesquelles R₃ représente H sont accessibles à partir de méthodes décrites dans la littérature, (par exemple : *Synthesis* (1984), **12**, 1032-1033 ; *Synth. Commun.* (1990), **20**(16), *2559-2564 ; J. Amer. Chem. Soc.* (1993), **115**(9), 3548-3557 ; *J. Med. Chem.* (1989), **32**(2), 391-396). *J. Amer. Chem. Soc.* (1995), **117**(11), 3308-3309). Les nitro-amines primaires de formule générale **(VI)** dans lesquelles R₃ représente H sont accessibles à partir de méthodes décrites dans la littérature (par exemple : *J. Chem. Soc.* (1947), 1487).

Lorsque X représente un radical phénylène et n et R₅ sont tels que définis ci-dessus, les amines / anilines de formule générale **(V)** dans lesquelles R₃ représente H sont accessibles par les méthodes illustrées dans le schéma 2.1 ci-après.

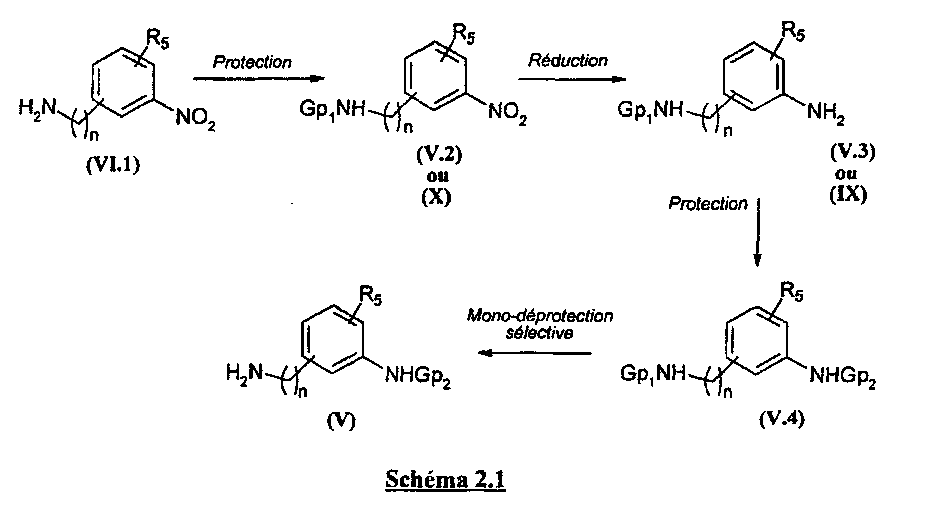

Les composés de formule générale **(V)**, schéma 2.1, sont préparés à partir des nitro-amines ou nitro-anilines de formule générale **(VI.1)**. La protection de la fonction amine ou aniline est effectuée par exemple, dans un solvant approprié tel que le dioxane ou le dichlorométhane ou l'acétonitrile en présence de Fmoc-Cl (*Tetrahedron Letters.*(1989), **30**(11), 1401-1404) ou (Boc)₂, ou de précurseurs d'autres groupes protecteurs (Gp) connus de l'homme du métier pour conduire aux composés de formule générale **(V.2)** (ou aux composés de formule générale **(X)**). La réduction de la fonction nitro des intermédiaires de formule générale **(V.2)** est généralement effectuée par hydrogénation catalytique, dans l'éthanol, en présence de Pd/C 10% ou par les autres méthodes précédemment décrites, pour conduire aux anilines **(V.3)** (ou aux composés de formule générale **(IX)**). La protection de la fonction aniline de formule générale **(V.3)** est effectuée, par exemple, en présence de Fmoc-Cl ou (Boc)₂ ou de précurseurs d'autres groupes protecteurs (Gp) connus de l'homme du métier, étant entendu que Gp₁ ≠ Gp₂, schéma 2.1. La dernière étape de la synthèse consiste à régénérer l'amine primaire par mono-déprotection, par exemple, quand Gp₁ = Fmoc, la déprotection est effectuée, par exemple, dans un solvant approprié tel que le DMF ou le dioxane, en présence d'une base telle que la pipéridine, la morpholine, la DMAP, ou la diisopropyléthylamine (*Tetrahedron Letters.*(1989), **30** (11), 1401-1404). Quand Gp₂ = Boc, ceux-ci sont déprotégés classiquement à l'aide d'acide trifluoroacétique ou de HCl, pour conduire finalement aux anilines mono-protégées de formule générale **(V)**.

Les composés de formule générale **(IX)** et **(X)** pour lesquels R₃ représente le radical 2-hydroxy-4,6-diméthoxyphénol, Gp représente le groupe Boc et n et R₅ sont tels que définis ci-dessus, sont préparés à partir des nitro-amines ou nitro-anilines de formule générale **(VI.1)**, schéma 6.1.

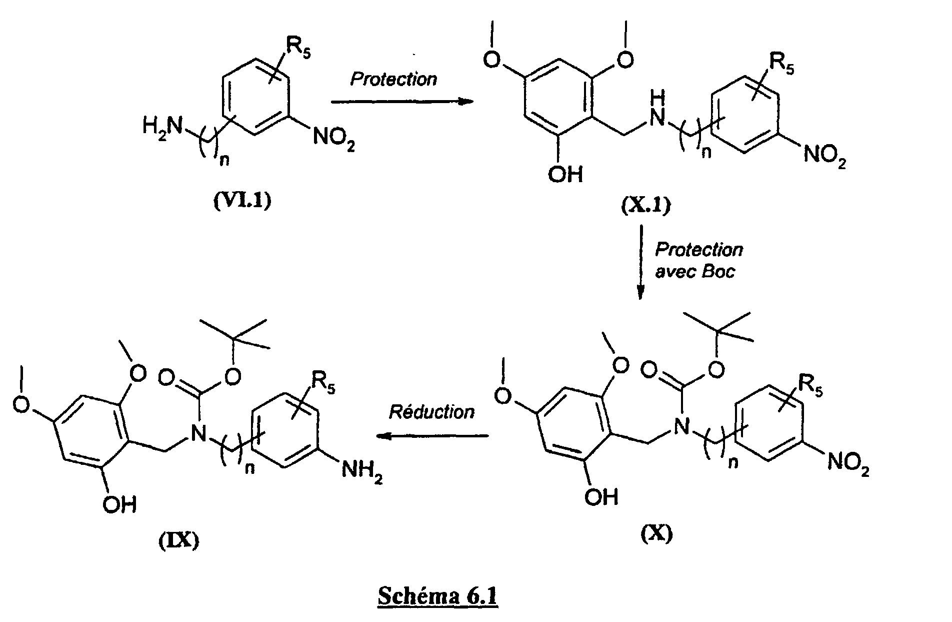

Les composés de formule générale **(IX)** et **(X)** sont préparés, schéma 6.1, par condensation de 2-hydroxy-4,6-diméthoxybenzaldéhyde avec une amine / aniline de formule générale **(VI.1)** en milieu réducteur. La réaction a lieu dans un solvant alcoolique tel que, par exemple, le méthanol, en présence d'un agent réducteur tel que, par exemple, NaBH₄ ou NaBH₃CN. La protection de l'amine secondaire formée est ensuite effectuée classiquement avec (Boc)₂ dans du dichlorométhane pour conduire aux composés de formule générale **(X)**. La réduction de la fonction nitro des composés de formule générale **(X)** est effectuée par hydrogénation catalytique, dans l'éthanol, en présence de Pd/C 10% pour conduire aux anilines **(IX)**.

Lorsque X représente un radical phénylène et R₅ représente un hétérocycle de 5 à 6 chaînons comprenant un atome d'azote (het) ou un groupe comportant un hétéroatome W (groupe noté Wαβ avec W = O, N ou S, α représentant alkyle et β n'existant pas lorsque W = O ou S ou β représentant H ou alkyle lorsque W = N), schémas 3.1 et 3.2, les amines / anilines de formule générale (X) non commerciales dans lesquelles R₃ représente H sont accessibles à partir de méthodes de la littérature (par exemple : *J. Med. Chem.* (1980), **23**, 973-975; *J. Med. Chem.* (1990), **33**, 633-641; *Chem.* *Heterocycl. Compd.* (EN). (1969), **5**, 683-687 ; *J. Org. Chem. USSR.* (EN) (1989), **3**, 599-600 ; *J. Med. Chem.* (1994), **37**, 467-475 ; *J. Med. Chem.* (1999), **42**, 4362-4379). Par exemple, les composés de formule générale **(X)** peuvent être préparés par les méthodes illustrées dans le schéma 3.1.

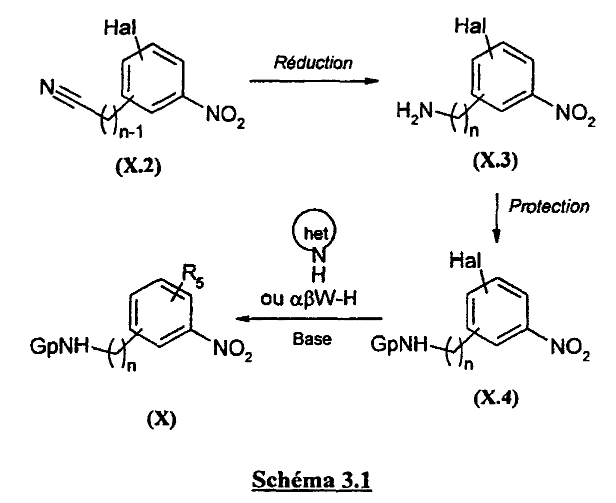

Les composés de formule générale **(X)**, schéma 3.1, dans laquelle n, het et Wαβ sont tels que définis ci-dessus, sont préparés à partir des halogéno-nitrobenzonitriles de formule générale **(X.2)**. La réduction de la fonction nitrile des intermédiaires de formule générale **(X.2)**, schéma 3.1, est effectuée, par exemple, dans un solvant approprié tel que l'éther ou le THF, en présence de diborane ou de LAH. Les halogéno-nitroanilines /aminés **(X.3)** formées sont ensuite protégée sous forme de Boc **(X.4)** ou d'autres groupes protecteurs (Gp) connus de l'homme du métier puis les composés de formule générale **(X.4)** sont soumis à une substitution nucléophile par un groupe hétérocyclique (het) dans un solvant comme le DMSO ou le DMF, en présence d'une base telle que K₂CO₃, KOH ou NaOH, pour conduire aux intermédiaires de formule générale **(X)**.

Alternativement, les composés de formule générale **(X)** tels que définis précédemment peuvent être préparés selon une méthode inspirée de celle représentée dans le schéma 3.2 ci-après :

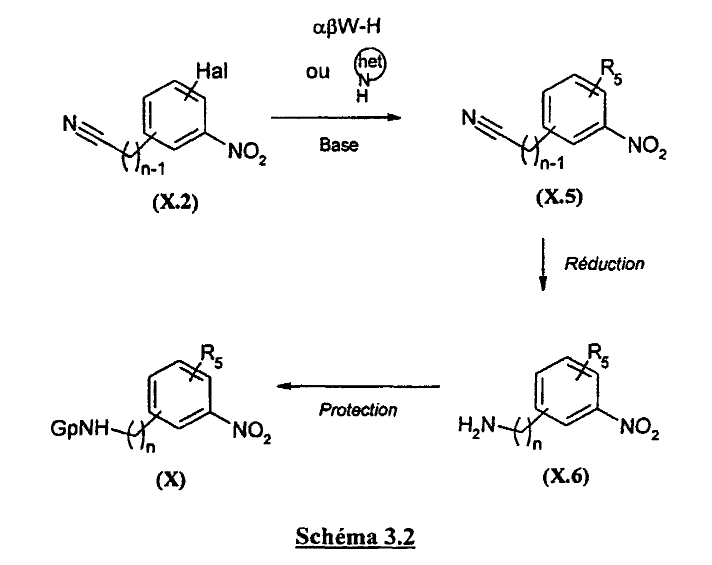

Les composés de formule générale **(X)**, schéma 3.2, dans laquelle R₅ représente un hétérocycle de 5 à 6 chaînons comprenant un atome d'azote (het) ou un groupe comportant un hétéroatome W (Wαβ tel que défini précédemment), sont préparés à partir des halogéno-nitrobenzonitriles de formule générale **(X.2)**. Les composés de formule générale **(X.2)** sont soumis à une substitution nucléophile par le réactif adéquat dans un solvant comme le DMSO ou le DMF, en présence d'une base telle que K₂CO₃, KOH ou NaOH, pour conduire aux intermédiaires de formule générale **(X.5)**.

La réduction de la fonction nitrile des intermédiaires de formule générale **(X.5)**, schéma 3.2, est effectuée, par exemple, dans un solvant approprié tel que l'éther ou le THF, en présence de diborane ou de LAH. Les halogéno-nitroanilines / amines **(X.6)** formées sont ensuite protégées sous forme de Boc ou d'autres groupes protecteurs (Gp) connus de l'homme du métier pour conduire finalement aux intermédiaires de formule générale **(X)**, schéma 3.2.

Les exemples suivants sont présentés pour illustrer les procédures ci-dessus et ne doivent en aucun cas être considérés comme une limite à la portée de l'invention.

### EXEMPLES

### Exemple 1 : chlorhydrate de N-{4-[[(2-thiényl)(imino)méthyl]amino] phényl}-1,2-dithiolane-3-pentanamide :

### 1.1) N-{4-[(1,1-diméthyléthoxy)carbonylamino]phényl}-1,2-dithiolane-3-pentanamide

A une solution de 2 g (9,694 mmol) d'acide (DL)-thioctique dans 40 ml de dichlorométhane, on ajoute successivement 1,84 g (8,81 mmol) de N-BOC-1,4-phénylène diamine, 1,6 ml de triéthylamine, 1,7 g (12,6 mmol) d'hydroxybenzotriazole, 4,82 g (25,2 mmol) de chlorhydrate de 1-(3-diméthylaminopropyl)-3-éthyl-carbodiimide et 1,6 ml de triéthylamine. Après avoir agité le mélange réactionnel une nuit à 25 °C, on dilue l'ensemble avec 100 ml d'eau et l'agitation est maintenue 30 minutes supplémentaires. Le produit est extrait à l'aide de 3 fois 100 ml de dichlorométhane. La solution organique est séchée sur sulfate de magnésium, filtrée et concentrée sous vide. La poudre rousse obtenue est suspendue dans l'éther (100 ml), filtrée et rincée par le même volume d'éther pour conduire à une poudre rose saumon avec un rendement de 80,5%. Point de fusion : 190-195 °C
RMN ¹H (DMSO d6, 400 MHz, δ) : 1,39 (m, 2H, CH₂) ; 1,57 (s, 9H, BOC) ; 1,61 (m, 4H, CH₂) ; 1,88 (m, 1H, CH₂) ; 2,27 (m, 2H, CH₂) ; 2,50 (m, 1H, CH₂) ; 3,15 (m, 2H, CH₂) ; 3,63 (m, 1H, -S-CH-) ; 7,34 (d, 2H, arom., J = 8,70 Hz) ; 7,45 (d, 2H, arom., J = 9,00 Hz) ; 9,24 (s, 1H, CONH) ; 9,77 (s, 1H, CONH-BOC).

### 1.2) N-(4-aminophényl)-1,2-dithiolane-3-pentanamide

Dans une solution de l'intermédiaire 1.1 (6,5 g ; 16,4 mmol) dans un mélange (200 ml) d'éther/éthanol/acétone/dichlorométhane (1/1/1/1), on fait passer un courant d'HCl gaz bulle à bulle à 0 °C. On laisse remonter à température ambiante toute la nuit. On fait passer un courant d'argon à travers la masse réactionnelle et on évapore les solvants à sec. Le résidu d'évaporation est ensuite versé dans 100 ml d'une solution saturée de NaHCO₃ froide et extrait par 3 x 100 ml de dichlorométhane. La phase organique est séchée sur sulfate de magnésium, filtrée et concentrée sous vide. On procède alors à une purification sur colonne de silice (éluant = heptane à 50 % acétate d'éthyle puis dichlorométhane à 5 % éthanol) pour conduire à un solide beige avec un rendement de 29%. Point de fusion : 55-60 °C.
RMN ¹H (DMSO d6, 400 MHz, δ) : 1,38 (m, 2H, CH₂) ; 1,57 (m, 4H, CH₂) ; 1,87 (m, 1H, CH₂) ; 2,22 (m, 2H, CH₂) ; 2,40 (m, 1H, CH₂) ; 3,18 (m, 2H, CH₂) ; 3,62 (m, 1H, -S-CH-) ; 4,78 (s, 2H, NH₂) ; 6,48 (d, 2H, arom., J = 8,64 Hz) ; 7,20 (d, 2H, arom., J = 8,64 Hz) ; 9,39 (s, 1H, CONH).

### 1.3) chlorhydrate de N-{4-[[(2-thiényl)(imino)méthyl]amino]phényl}-1,2-dithiolane-3-pentanamide :

L'intermédiaire 1.2 (0,703 g ; 2,37 mmol) est dissous dans du 2-propanol (15 ml) et l'on ajoute 1,014 g d'iodhydrate de S-méthyl-2-thiophène thiocarboximide (3,56 mmol) (*Ann. Chim.* (1962), **7**, 303-337). Après chauffage à 60 °C pendant 15 heures, le mélange réactionnel est concentré à sec sous vide. Le résidu est repris dans du dichlorométhane et une solution aqueuse saturée de NaHCO₃. Après décantation, la phase organique est lavée successivement par 50 ml d'une solution saturée de NaHCO₃, d'eau et de saumure. La solution organique est séchée sur sulfate de magnésium, filtrée et évaporée sous pression réduite. On dissout ensuite la base libre dans 30 ml de dichlorométhane et la solution est refroidie à l'aide d'un bain de glace avant l'addition goutte à goutte de 6,3 ml d'une solution 1N HCl dans l'éther éthylique anhydre. Après 15 heures d'agitation à 25 °C, les cristaux obtenus sont filtrés et rincés à l'éther diéthylique pour obtenir après séchage 1,6 g d'un produit solide beige clair avec un rendement de 77%. Point de fusion : 258,7-258,9 °C
RMN ¹H (DMSO d6, 400 MHz, δ) : 1,43 (m, 2H, CH₂) ; 1,62 (m, 4H, CH₂) ; 1,88 (m, 1H, CH₂) ; 2,38 (m, 3H, CH₂) ; 3,18 (m, 2H, CH₂) ; 3,63 (m, 1H, -S-CH-) ; 7,20-8,20 (m, 7H, arom.) ; 8,79 (s large, 1H, NH⁺) ; 9,78 (s large, 1H, NH⁺) ; 10,36 (s, 1H, CONH) ; 11,49 (s large, 1H, NH⁺).
IR : ν_{C=N} (amidine) : 1580 cm⁻¹ ; ν_{C=O} (amide) : 1659 cm⁻¹.

### Exemple 2: N-{2-{4[[(2-thiényl)(imino)méthyl]amino]phényl}éthyl}-1,2-dithiolane-3-pentanamide :

### 2.A. Méthode selon la première approche :

Le protocole expérimental utilisé est le même que celui décrit pour le composé 1. Solide jaune. Point de fusion : 146-148 °C.
RMN ¹H (DMSO d6, 400 MHz, δ) : 1,32 (m, 2H, CH₂) ; 1,60 (m, 4H, CH₂) ; 1,88 (m, 1H, CH₂) ; 2,07 (t, 2H, CH₂, J = 7,36 Hz) ; 2,41 (m, 1H, CH₂) ; 2,65 (m, 2H, CH₂) ; 3,10-3,30 (m, 4H, CH₂) ; 3,60 (m, 1H, -S-CH-) ; 6,33 (s large, 2H, NH₂ amidine) ; 6,78 (d, 2H, arom., J = 8,04 Hz) ; 7,08 (m, 1H, thiophène) ; 7,12 (d, 2H, arom., J = 8,16 Hz) ; 7,60-7,80 (m, 2H, thiophène) ; 7,81 (t, 1H, CONH J = 5,56 Hz).
IR : ν_{C=N} (amidine) : 1590 cm⁻¹ ; ν_{C=O} (amide) : 1629 cm⁻¹.

### 2.B. Méthode selon la deuxième approche :

Alternativement, la synthèse du composé de l'exemple 2 peut être effectuée selon la méthode illustrée par les schémas 3 (avec Gp = Boc et R₃ = 2-hydroxy-4,6-diméthoxybenzyle) et 6.

### 2.B.1) 3,5-diméthoxy-2-({[2-(4-nitrophényl)éthyl]amino}méthyl)phénol :

Dans un ballon contenant 200 ml de MeOH anhydre, sous atmosphère inerte, on ajoute successivement 9,0 g (49,4 mmol) de 4,6-diméthoxysalicaldéhyde, 11,6 g (54,3 mmol) de chlorhydrate de 4-nitrophénéthylamine et 7,5 ml de triéthylamine. Le mélange réactionnel est agité vigoureusement pendant 15 heures avant l'addition, par portions, de 2,1 g (55,5 mmol) de NaBH₄. L'agitation est maintenue 10 heures supplémentaires avant addition de 10 ml d'eau. Après un quart d'heure, le mélange réactionnel est extrait avec 2 fois 100 ml de CH₂Cl₂. La phase organique est lavée successivement avec 50 ml d'eau et 50 ml de saumure, séchée sur sulfate de sodium, filtrée et concentrée sous vide. Le résidu est alors purifié sur une colonne de silice (éluant : CH₂Cl₂EtOH : 20/1). On obtient une huile orange avec un rendement de 58%.

### 2.B.2) 2-hydroxy-4,6-diméthoxybenzyl[2-(4-nitrophényl)éthyl]carbamate de tert-butyle :

Le protocole expérimental utilisé est le même que celui décrit pour l'intermédiaire 7.B.1, l'intermédiaire 2.B.1 remplaçant la p-nitrobenzylamine On obtient un solide blanc avec un rendement de 60%. Point de fusion : 133,5-134,4°C

### 2.B.3) 2-(4-aminophényl)éthyl(2-hydroxy-4,6-diméthoxybenzyl)carbamate de tert-butyle:

Le protocole expérimental utilisé est le même que celui décrit pour l'intermédiaire 7.B.2, l'intermédiaire 2.B.2 remplaçant l'intermédiaire 7.1. On obtient une huile jaune clair avec un rendement de 90%.

### 2.B.4) 2(4-{[(amino(2-thiényl)méthylidène]amino}phényl)éthylcarbamate de tert-butyle:

Le protocole expérimental utilisé est le même que celui décrit pour l'intermédiaire 1.3, l'intermédiaire 2.B.3 remplaçant l'intermédiaire 1.2. Ces conditions provoquent la N-débenzylation pour conduire à l'amine primaire mono protégée avec le groupement Boc. On obtient un solide jaune avec un rendement de 79%. Point de fusion : 144°C

### 2.B.5) N'-[4-(2-aminoéthyl)phényl]-2-thiophènecarboximidamide :

Le protocole expérimental utilisé est le même que celui décrit pour l'intermédiaire 1.2, l'intermédiaire 2.B.4 remplaçant l'intermédiaire 1.1. On obtient un solide blanc avec un rendement de 79%. Point de fusion : 169.2-170.5°C.

### 2.B.6) N-{2-{4[[(2-thiényl)(imino)méthyl]amino]phényl}éthyl}-1,2-dithiolane-3-pentanamide :

Le protocole expérimental utilisé est le même que celui décrit pour l'intermédiaire 1.1, l'intermédiaire 2.B.5 remplaçant la N-BOC-1,4-phénylène diamine. On obtient un solide jaune avec un rendement de 79%. Point de fusion : 146-148 °C.
RMN ¹H (DMSO d6, 400 MHz, δ) : 1,32 (m, 2H, CH₂) ; 1,60 (m, 4H, CH₂) ; 1,88 (m, 1H, CH₂) ; 2,07 (t, 2H, CH₂, J = 7,36 Hz) ; 2,41 (m, 1H, CH₂) ; 2,65 (m, 2H, CH₂) ; 3,10-3,30 (m, 4H, CH₂) ; 3,60 (m, 1H, -S-CH-) ; 6,33 (s large, 2H, NH₂ amidine) ; 6,78 (d, 2H, arom., J = 8,04 Hz) ; 7,08 (m, 1H, thiophène) ; 7,12 (d, 2H, arom., J = 8,16 Hz) ; 7,60-7,80 (m, 2H, thiophène) ; 7,81 (t, 1H, CONH J = 5,56 Hz).
IR : ν_{C=N} (amidine) : 1590 cm⁻¹ ; ν_{C=O} (amide) : 1629 cm⁻¹.

### 2.C. Autre méthode selon la deuxième approche:

Une autre synthèse illustrée par le schéma 3 peut également être employée, avec Gp = Boc. Le protocole expérimental est dans ce cas analogue à celui décrit dans la procédure 7.B ci-après, la 4-nitrophénéthylamine remplaçant la p-nitrobenzylamine.

### Exemple 3 : chlorhydrate de N-{2-{4[[(2-thiényl)(imino)méthyl}amino] phényl}éthyl}-1,2-dithiolane-3-acétamide:

### 3.A. Méthode selon la première approche :

Le protocole expérimental utilisé est le même que celui décrit pour le composé 1, l'acide trisnorlipoïque [acide 2-(1,2-dithiolan-3-yl)acétique] (préparé selon *Tetrahedron Letters,* (1997), **38**, 33, 5785) remplaçant l'acide lipoïque. Solide jaune.
RMN ¹H (DMSO d6, 400 MHz, δ) : 1,91 (m, 1H, CH₂) ; 2,30-2,60 (m, 3H, CH₂) ; 2,70-2,90 (m, 2H, CH₂); 3,17 (m, 2H, CH₂); 3,40 (m, 2H, CH₂); 3,93 (m, 1H, -S-CH-); 7,30-7,50 (m, 5H, arom.) ; 8,10-8,30 (m, 3H, arom. + CONH); 8,86 (s large, 1H, NH⁺); 9,80 (s large, 1H, NH⁺) ; 11,50 (s large, 1H, NH⁺).
MS : MH+ = 392,1.

### 3.B. Méthode selon la deuxième approche:

Alternativement, la synthèse du composé de l'exemple 3 peut être effectuée selon la méthode illustrée par les schémas 3 (avec Gp = Boc et R₃ = 2-hydroxy-4,6-diméthoxybenzyle) et 6. Le protocole expérimental utilisé est le même que celui décrit dans la procédure 2.B, l'acide trisnorlipoïque [acide 2-(1,2-dithiolan-3-yl)acétique] (préparé selon *Tetrahedron Letters,* (1997), **38**, 33, 5785) remplaçant l'acide lipoïque. Solide jaune.

### 3.C. Autre méthode selon la deuxième approche :

Une autre synthèse illustrée par le schéma 3 peut également être employée, avec Gp = Boc. Le protocole expérimental est dans ce cas analogue à celui décrit dans la procédure 7.B ci-après, la nitrophénéthylamine remplaçant la p-nitrobenzylamine et l'acide trisnorlipoïque [acide 2-(1,2-dithiolan-3-yl)acétique] (préparé selon *Tetrahedron Letters,* (1997), **38**, 33, 5785) remplaçant l'acide lipoïque.

### Exemple 4 : N-[4-(6-amino-4-méthyl-2-pyridinyl)buiyl]-1,2-dithiolane-3-pentanamide :

### 4.1) 6-(2,5-diméthyl-1H-pyrrol-1-yl)-4-methyl-2-pyridinebutanamine

Sous atmosphère d'argon, on dissout 1 g (5 mmol) de 2-(2,5-diméthyl-1H-pyrrol-1-yl)-4,6-diméthylpyridine (préparé à partir de la 6-amino-2,4-lutidine selon *J. Chem. Soc., Perkin Trans.,* 1984, **12**, 2801) dans 10 ml d'éther éthylique anhydre et 1,132 ml (7,5 mmol) de N,N,N,N-tétraméthyléthylènediamine (TMEDA). Le mélange réactionnel est refroidi à -20 °C et on ajoute goutte à goutte 2,4 ml (6 mmol) d'une solution 2,5 M de BuLi dans l'hexane. Après 5 heures à -20 °C, le mélange réactionnel est refroidi à -45 °C et on ajoute 1,68 g (6 mmol) de 1-(3-bromopropyl)-2,2,5,5-tétraméthyl-1-aza-2,5-disilacyclopentane et on laisse remonter à température ambiante toute la nuit. On ajoute 50 ml d'une solution saturée de chlorure d'ammonium et l'ensemble est agité 2 heures à 25°C. La phase organique est décantée et lavée successivement par 40 ml d'eau et 40 ml de saumure, séchée sur sulfate de magnésium, filtrée et concentrée sous vide. Le résidu obtenu est purifié sur une colonne de silice (éluant = dichlorométhane à 5 % éthanol) pour conduire à une huile jaune avec un rendement de 62%.
RMN ¹H (CDCl₃, 400 MHz, δ) : 1,52 (m, 2H, CH₂) ; 1,78 (m, 2H, CH₂) ; 2,11 (s, 6H, 2 x CH₃ pyrrole) ; 2,39 (s, 3H, CH₃ pyridine) ; 2,72 (t, 2H, CH₂ J = 7,02 Hz) ; 2,79 (t, 2H, CH₂ J = 7,62 Hz) ; 5,87 (s, 2H, pyrrole) ; 6,85 (s, 1H, pyridine) ; 6,98 (s, 1H, pyridine).

### 4.2) N{4-[6-(2,5-diméthyl-1H pyrrol-1-yl)-4-méthyl-2 pyridinyl]butyl}-1,2-dithiolane-3-pentanamide

Le protocole expérimental utilisé est le même que celui décrit pour l'intermédiaire 1.1, l'intermédiaire 4.1 remplaçant la N-BOC-1,4-phénylènediamine. On obtient une huile jaune.
RMN ¹H (CDCl₃, 400 MHz, δ): 1,30-2,00 (m, 11H, CH₂) ; 2,07 (m, 2H, CH₂) ; 2,11 (s, 6H, 2 x CH₃ pyrrole) ; 2,40 (s, 3H, CH₃ pyridine) ; 2,45 (m, 1H, CH₂) ; 2,82 (m, 2H, CH₂) ; 3,10-3,30 (m, 4H, CH₂) ; 3,57 (m, 1H, -S-CH-) ; 5,87 (s, 2H, pyrrole) ; 5,94 (s large, 1H, CONH) ; 6,87 (s, 1H, pyridine) ; 6,99 (s, 1H, pyridine).
MS : MH+ = 446,2

### 4.3) N-[4-(6-amino-4-méthyl-2 pyridinyl)butyl]-1,2-dithiolane-3-pentanamide

On dissout l'intermédiaire 4.2 (1,53 g ; 3,43 mmol) dans 55 ml d'éthanol additionné de 18 ml d'eau et on ajoute 1,2 g (17,2 mmol) de chlorhydrate d'hydroxylamine. Le mélange réactionnel est chauffé à reflux pendant 24 heures. Après retour à 25 °C, l'ensemble est dilué par 20 ml d'une solution saturée de bicarbonate de sodium et le produit est extrait par 100 ml de dichlorométhane. Après décantation, la solution organique est lavée successivement par 40 ml d'une solution saturée de bicarbonate de sodium et 40 ml de saumure, séchée sur sulfate de magnésium, filtrée et concentrée sous vide. Le résidu d'évaporation est purifié sur colonne de silice (éluant = dichlorométhane à 2,5 % éthanol) pour conduire à une huile jaune avec un rendement de 76 %.
RMN 1H (CDCl₃, 400 MHz, δ) : 1,30-1,80 (m, 10H, CH₂) ; 1,89 (m, 1H, CH₂) ; 2,16 (m, 2H, CH₂) ; 2,19 (s, 3H, CH₃ pyridine) ; 2,40 (m, 1H, CH₂) ; 2,58 (m, 2H, CH₂) ; 3,05-3,30 (m, 4H, CH₂) ; 3,56 (m, 1H, -S-CH-) ; 4,35 (s large, 2H, NH₂); 5,80 (s large, 1H, CONH) ; 6,17 (s, 1H, pyridine) ; 6,35 (s, 1H, pyridine).
IR : ν_{C=O} (amide) : 1637 cm⁻¹.

### Exemple 5 : fumarate de N-[4-(6-amine-4-méthyl-2-pyridinyl)butyl]-1,2-dithiolane-3-acétamide :

Le protocole expérimental utilisé est le même que celui décrit pour le composé 4. l'acide trisnorlipoique [ou acide 2-(1,2-dithiolan-3-yl)acétique] (préparé selon *Tetrahedron Letters,* 1997, **38**, 33, 5785) remplaçant l'acide lipoïque. La base libre est alors salifiée à l'aide d'acide fumarique dans un mélange de solvant acétone/méthyl éthyl cétone (50/50). On obtient le fumarate sous forme d'une poudre crème avec un rendement de 14,8 %.
RMN ¹H (DMSO d6, 400 MHz, δ) : 1,40 (m, 2H, CH₂) ; 1,57 (m, 2H, CH₂) ; 1,92 (m, 1H, CH₂) ; 2,15 (s, 3H, CH₃ pyridine) ; 2,30-2,70 (m, 5H, CH₂) ; 3,03-3,18 (m, 4H, CH₂) ; 3,94 (m, 1H, -S-CH-) ; 6,17 (s, 1H, pyridine) ; 6,26 (s, 1H, pyridine) ; 6,00-6,60 (s large, 2H, -CO₂H acide fumarique) ; 6,60 (s, 2H, CH=CH, acide fumarique) ; 7,90 (s large, 1H, CONH).
IR : v_{C=O} (amide) : 1649 cm⁻¹.
MS : MH+ = 326,2

### Exemple 6 : N-(4-{[amino(2-thiényl)méthylidène]amino}phényl)-2-(1,2-dithiolan-3-yl)acétamide :

Le protocole expérimental utilisé est le même que celui décrit pour le composé 1, l'acide trisnorlipoïque [ou acide 2-(1,2-dithiolan-3-yl)acétique] (préparé selon *Tetrahedron Letters,* (1997), **38**, 33, 5785) remplaçant l'acide lipoïque. Mousse jaune.
RMN ¹H (DMSO d6, 400 MHz, δ) : 2,00 (m, 1H, CH₂) ; 2,40-2,80 (m, 3H, CH₂) ; 3,00-3,30(m,2H,CH₂); 4,10(m,1H,)-S-CH-) ; 6.50-6.80 (s large, 2H, NH⁺) ; 6.80-7.10 (m, 3H, arom.) ; 7,50-7,80 (m, 4H, arom.) ; 9.93 (s, 1H, CONH).
MH+ = 364,1.

### Exemple 7 : N-(4-{[amino(2-thiényl}méthylidène]amino}benzyl)-5-(1,2-dithiolan-3-yl)pentanamide :

### 7.A. Méthode selon la première approche:

Le protocole expérimental utilisé est le même que celui décrit pour le composé 1, le 4-(aminométhyl)phénylcarbamate de tert-butyle remplaçant la N-BOC-1,4-phénylène diamine. Solide blanc. Point de fusion : 151,9-152,1 °C.

### 7.B. Méthode selon la deuxième approche :

### 7.B.1. 4-nitrobenzylcarbamate de tert-butyle:

On dissout 5,66 g (30,0 mmol) de chlorhydrate de p-nitrobenzylamine dans un mélange de 100 ml de dichlorométhane et 9,2 ml de triéthylamine. L'ensemble est refroidi à l'aide d'un bain de glace avant l'addition en plusieurs portions de 7,2 g (33,0 mmol) de (Boc)₂O. Le mélange réactionnel est agité à 23 °C pendant 12 heures et versé dans un mélange eau-glace. La phase organique est décantée, lavée successivement avec 20 ml d'eau et 20 ml de saumure. Après séchage sur sulfate de sodium, filtration et concentration sous vide, on obtient un solide blanc après trituration avec de l'éther isopropylique avec un rendement de 67,4%. Point de fusion : 107,8 °C.

### 7.B.2. 4-aminobenzylcarbamate de tert-butyle :

Dans un autoclave en acier inox équipé d'un barreau magnétique, on introduit une solution de l'intermédiaire 7.B.1 (5,1 g; 20,2 mmol) dans 66 ml d'un mélange dichlorométhane, d'acétate d'éthyle et de THF (1 ml / 60 ml / 5 ml) ainsi que 1,0 g de Pd/C à 10%. Le mélange réactionnel est agité sous pression d'hydrogène (1,5 bar) pendant 12 heures à une température de 20 °C. Le Pd/C est ensuite éliminé par filtration et le filtrat est concentré sous vide. Une fois le résidu d'évaporation purifié par trituration avec de l'éther isopropylique, on obtient une poudre gris blanc avec un rendement de 42%. Point de fusion : 74,4 °C.

### 7.B.3. 4-{[amino(2-thiényl)méthylidène]amino}benzylcarbamate de tert-butyle :

Le protocole expérimental utilisé est le même que celui décrit pour l'intermédiaire 1.3, l'intermédiaire 7.B.2 remplaçant l'intermédiaire 1.2. On obtient une huile orange avec un rendement de 99%.
RMN ¹H (DMSO d6, 400 MHz, δ): 1,40 (s, 9H, 3xCH₃) ; 4,19 (d, 2H, CH₂, J = 6,00 Hz) ; 7,30-7,40 (m, 5H, arom.) ; 7,46 (t, 1H, CONH , J = 6,00 Hz) ; 8,10-8,20 (m, 2H, thiophène) ; 9,00-10,00 (s large, 2H, NH₂ amidine); 11,10-11,40 (s large, 1H, HI).
MH+ = 332,2.

### 7.B.4. N'-[4-(aminométhyl)phényl]-2-thiophènecarboximidamide :

Le protocole expérimental utilisé est le même que celui décrit pour l'intermédiaire 1.2, le l'intermédiaire 7.B.3 remplaçant l'intermédiaire 1.1. On obtient un solide blanc avec un rendement de 92%. Point de fusion : 241,1-241,6 °C

### 7.B.5. N-(4-{[amino(2-thiényl)méthylidène]amino}benzyl)-5-(1,2-dithiolan-3-yl)pentanamide :

Le protocole expérimental utilisé est le même que celui décrit pour l'intermédiaire 1.1, l'intermédiaire 7.B.4, remplaçant la N-BOC-1,4-phénylène diamine. On obtient un solide blanc avec un rendement de 40%. Point de fusion : 151,9-152,1 °C.

### Exemple 8 : N-(5-{[amino(2-thiényl)méthylidène]amino}-2-méthoxyphényl)-5-(1,2-dithiolan-3-yl)pentanamide :

### (Préparé par une méthode selon la première approche)

### 8.1. 5-(1,2-dithiolan-3-yl)-N-(2-méthoxy-5-nitrophényl)pentanamide :

A une solution de 4,12 g (21,0 mmol) d'acide (DL)-thioctique dans 50 ml de DMF, on ajoute successivement 3,36 g (21,0 mmol) de 2-méthoxy-5-nitroaniline, de la triéthylamine (6,0 ml), 3,0 g (22,0 mmol) d'hydroxybenzotriazole et 4,21 g (22,0 mmol) de chlorhydrate de 1-(3-diméthylaminopropyl)-3-éthyl-carbodiimide. Après avoir agité le mélange réactionnel pendant 18 heures à 80 °C, on dilue l'ensemble avec 400 ml d'eau et l'agitation est maintenue 30 minutes supplémentaires. Le produit est extrait à l'aide de 3 fois 200 ml de dichlorométhane. La solution organique est séchée sur sulfate de magnésium, filtrée et concentrée sous vide. Le solide obtenu est filtré et rincé à éther diéthylique pour obtenir après séchage 2,6 g d'un produit solide jaune (rendement de 37%.). Point de fusion : 116,7-117,1 °C.

### 8.2. N-(5-amino-2-méthoxyphényl)-5-(1,2-dithiolan-3-yl)pentanamide:

A une solution de 2,6 g (13,20 mmol) de l'intermédiaire 8.1 dans 40 ml d'éthanol, on ajoute successivement 10 ml d'une solution aqueuse de chlorure d'ammonium saturée et 12,0 g (0,183 mol) d'Indium en poudre. Le milieu réactionnel est alors porté à reflux pendant 4 heures et demie (*Synlett* (1998), **9**, 1028). L'ensemble est refroidi à température ambiante et filtré sur célite. Le filtrat est alcalinisé à pH 10 avec une solution d'hydroxyde de sodium à 50%. Le produit réduit est extrait à l'aide de 4 fois 150 ml de dichlorométhane. La solution organique est séchée sur sulfate de magnésium, filtrée et concentrée sous vide pour conduire à une huile marron. On dissout l'huile dans 50 ml d'acétate d'éthyle et l'ensemble est refroidi à 0 °C à l'aide d'un bain de glace. Une solution de bicarbonate de potassium à 10% dans l'eau est alors ajoutée goutte à goutte. Après avoir agité le mélange réactionnel environ 10 minutes à 0 °C, on ajoute la solution d'Iode (0,8 g dans 10 ml de l'acétate d'éthyle) goutte à goutte jusqu'à ce que la coloration d'iode persiste. Le produit est extrait à l'aide de 4 fois 100 ml d'acétate d'éthyle et la solution organique est séchée sur sulfate de magnésium, filtrée et concentrée sous vide. On procède alors à une purification sur colonne de silice (éluant = 5% d'éthanol dans du dichlorométhane) pour conduire à une huile marron (1,0 g ; rendement de 63%).
MH⁺ =327,20.

### 8.3. N-(5-{[amino(2-thiényl)méthylidène]amino}-2-méthoxyphényl)-5-(1,2-dithiolan-3-yl)pentanamide :

MH⁺=436,10.

### Exemple 9 : N-[5-{[amino(2-thiényl)méthylidène]amino}-2-(diméthylamino)benzyl]-5-(1,2-dithiolan-3-yl)pentanamide:

### (Préparé par une méthode selon la deuxième approche)

### 9.1. 2-(diméthylamino)-5-nitrobenzonitrile :

Sous atmosphère d'argon, on dissout 1,66 g (10,0 mmol) de 2-fluoro-5-nitrobenzonitrile, 1,22 g (15,1 mmol) de chlorhydrate de diméthylarnine et 3,46 g (25,1 mmol) de bicarbonate de potassium dans du DMF (30 ml) puis le milieu réactionnel est porté à une température de 80 °C pendant 18 heures. Le mélange réactionnel est refroidi à 0 °C et on ajoute de l'eau glacée. Le mélange réactionnel est extrait avec de l'acétate éthyle et la phase organique est lavée successivement avec 50 ml d'eau et 50 ml de saumure, séchée sur sulfate de magnésium, filtrée et concentrée sous vide. Le résidu d'évaporation est purifié par trituration avec de l'éther isopropylique et le solide obtenu est filtré et rincé à l'isopentane pour obtenir après séchage 2,0 g d'un produit solide jaune (rendement de 100%). Point de fusion : 109-110,5 °C.

### 9.2. 2-(aminométhyl)-N,N-diméthyl-4-nitroaniline:

L'intermédiaire 9.1 (1,5 g ; 7,85 mmol) est mis en solution sous atmosphère d'argon dans du THF (40 ml). Une solution de diborane (16 ml, 1M dans THF) est ajoutée puis le milieu réactionnel porté à reflux pendant 6 heures. Du méthanol (30 ml) est additionné puis HCl gaz est barboté à travers le mélange pendant 15 minutes. Le milieu réactionnel est évaporé à sec et repris avec une solution de bicarbonate de sodium. On extrait au dichlorométhane puis la phase organique est lavée successivement par 50 ml d'eau et 50 ml de saumure, séchée sur sulfate de magnésium, filtrée et concentrée sous vide. Le résidu d'évaporation est purifié par cristallisation dans un mélange d'éther isopropylique et de dichlorométhane pour conduire à un produit solide jaune avec un rendement de 82%. Point de fusion : 196-200 °C.

### 9.3. 2-(diméthylamino)-5-nitrobenzylcarbamate de tert-butyle:

Le protocole expérimental utilisé est le même que celui décrit pour l'étape 7.B.1 de l'exemple 7, l'intermédiaire 9.2 remplaçant le chlorhydrate de p-nitrobenzylamine. On obtient un solide jaune avec un rendement de 100%. Point de fusion : 101-102 °C.

### 9.4. 5-amino-2-(diméthylamino)benzylcarbamate de tert-butyle :

Le protocole expérimental utilisé est le même que celui décrit pour l'étape 7.B.2 de l'exemple 7, l'intermédiaire 9.3 remplaçant l'intermédiaire 7.B.1. On obtient une huile marron avec un rendement de 15%. MH⁺ = 266,20.

### 9.5. 5-{[amino(2-thiényl)méthylidène]amino}-2(diméthylamino)benzylcarbamate de tert-butyle

Le protocole expérimental utilisé est le même que celui décrit pour l'étape 1.3 de l'exemple 1, l'intermédiaire 9.4 remplaçant l'intermédiaire 1.2. On obtient un solide jaune avec un rendement de 64%. Point de fusion : 175,8°C-177,0°C.

### 9.6. N'-[3-(aminométhyl)-4-(diméthylamino)phényl]-2-thiophènecarboximidamide :

Le protocole expérimental utilisé est le même que celui décrit pour l'étape 1.2 de l'exemple 1, l'intermédiaire 9.5 remplaçant l'intermédiaire 1.1. On obtient un solide jaune avec un rendement de 66%. Point de fusion : 169,0-170,0 °C.

### 9.7. N-[5-{[amino(2-thiényl)méthylidène]amino}-2-(diméthylamino)benzyl]-5-(1,2-dithiolan-3-yl)pentanamide:

Le protocole expérimental utilisé est le même que celui décrit pour l'étape 1.1 de l'exemple 1, l'intermédiaire 9.6 remplaçant la N-BOC-1,4-phénylènediamine. On obtient un solide blanc avec un rendement de 67%. Point de fusion : 183,5-185,0 °C.

### Exemple 10 : N-[5-{[amino(2-thiényl)méthylidène]amino}-2-(1H-pyrrol-1-yl)benzyl]-5-(1,2-dithiolan-3-yl)pentanamide :

Le protocole expérimental utilisé est le même que celui décrit pour le composé de l'exemple 9, le pyrrole remplaçant le chlorhydrate de diméthylamine dans la première étape. On obtient un solide blanc avec un rendement de 72%. Point de fusion: 156,1-157,6 °C.

### Etude pharmacologique des produits de l'invention

### Etude des effets sur la NO synthase constitutive neuronale de cervelet de rat

L'activité inhibitrice des produits de l'invention est déterminée par la mesure de leur effets sur la transformation par la NO synthase de la [³H]L-arginine en [³H]L-citrulline en accord avec la méthode modifiée de Bredt et Snyder (*Proc. Natl. Acad. Sci. USA,* (1990) **87**: 682-685). Des cervelets de rats Sprague-Dawley (300g - Charles River) sont rapidement prélevés, disséqués à 4°C et homogénéisés dans un volume de tampon d'extraction (HEPES 50 mM, EDTA 1 mM, pH 7,4, pepstatin A 10 mg/ml, leupeptine 10 mg/ml). Les homogénats sont ensuite centrifugés à 21000 g pendant 15 min à 4 °C. Le dosage se fait dans des tubes à essai en verre dans lesquels sont distribués 100 µl de tampon d'incubation contenant 100 mM d'HEPES (pH 7,4), 2 mM d'EDTA, 2,5 mM de CaCl₂, 2 mM de dithiotréitol, 2 mM de NADPH réduit et 10 µg/ml de calmoduline. On ajoute 25 µl d'une solution contenant 100 nM de [³H]L-arginine (activité spécifique : 56,4 Ci/mmole, Amersham) et 40 µM de L-arginine non radioactive. La réaction est initiée en ajoutant 50 µl d'homogénat, le volume final étant de 200 µl (les 25 µl manquants sont soit de l'eau, soit le produit testé. Après 15 min, la réaction est stoppée avec 2 ml de tampon d'arrêt (20 mM d'HEPES, pH 5,5, 2 mM d'EDTA). Après passage des échantillons sur une colonne de 1 ml de résine DOWEX, la radioactivité est quantifiée par un spectromètre à scintillation liquide.

Les composés des exemples 1, 2, 3, 4, 5 décrits ci-dessus présentent une Cl₅₀ inférieure à 4,5 µM.

### Etude des effets sur le stress oxidatif induit par le glutamate sur des cellules en culture (HT-22).

L'activité inhibitrice des produits de l'invention est déterminée par la mesure de leur capacité à protéger les cellules d'une lignée hippocampale de souris (HT-22) d'un stress oxidatif provoqué par le glutamate. La biosynthèse de glutathion, élément essentiel de détoxification cellulaire des radicaux libres, nécessite le transport actif de cystine à l'intérieur de la cellule. Le glutamate en s'opposant à la pénétration de la cystine provoque une réduction du taux de glutathion qui conduit à la mort de la cellule par stress oxidatif (Davis, J.B. and Maher, P., *Brain Res.,* (1994) **652**: 169-173; Murphy, T.H. et al., *Neuron,* (1989) **2**: 1547-1558). Les cellules sont cultivées à 37 °C dans un milieu DMEM additionné de 10% de sérum de veau foetal. Les essais sont réalisés dans des plaques 96 puits contenant 5000 cellules par puits. Le glutamate (5 mM) est ajouté au milieu contenant ou non les produits à tester. La viabilité cellulaire est testée après 24 h par la méthode du MTT (Hansen, M.B. et al., *J Immunol.Methods* (1989), **119**, 203-210). La capacité des composés à protéger les cellules de l'action toxique du glutamate est estimée en CE₅₀, calculée par rapport à la viabilité de cellules non soumises à l'action du glutamate considérée comme viabilité 100%.

Les composés des exemples 1, 2, 3, 5 décrits ci-dessus présentent une CE₅₀ inférieure à 30 µM.

## Revendications

1. Produit de formule générale **(I)**, **caractérisée en ce qu'**elle comprend les produits de sous-formules **(I)a** et **(I)b** 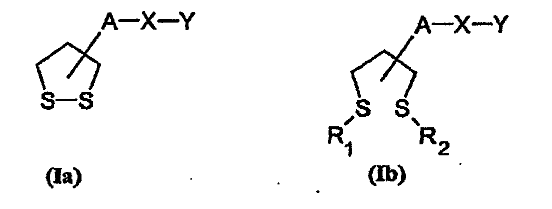 dans lesquelles
R₁ et R₂ représentent indépendamment un atome d'hydrogène ou un radical alkyle linéaire ou ramifié de 1 à 6 atomes de carbone ;
A représente l'un des radicaux (CH₂)ₘ-NR₃-CO(CH₂)ₙ-, -(CH₂)ₘ-CO-NR₃-(CH₂)ₙ-, -(CH₂)ₘ-NR₃-(CH₂)ₙ-, -(CH₂)ₘ-CO-NR₃-(CH₂)ₚ-NR₄-(CH₂)ₙ-, -(CH₂)ₘ-NR₃-CO-NR₄-(CH₂)ₙ- ou -(CH₂)ₘ-,
m et n étant des entiers de 0 à 6,
p étant un entier de 2 à 6,
et R₃ et R₄ représentant indépendamment un atome d'hydrogène ou un radical alkyle linéaire ou ramifié de 1 à 6 atomes de carbone ;
X représente un radical 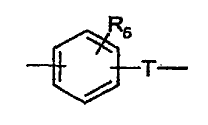 dans lequel le groupe T, qui est attaché au groupe Y, représente un radical -(CH₂)ᵢ- dans lequel i représente un entier compris entre 0 et 6, et R₅ représente un atome d'hydrogène, un radical alkyle linéaire ou ramifié ayant de 1 à 6 atomes de carbone ou un radical -(CH₂)ₘ,-Q dans lequel Q représente un atome halogène ou un radical hydroxy, cyano, amino, alkoxy, alkylthio, aikylamino ou dialkylamino, ou encore R₅ représente un hétérocycle de 5 à 6 chaînons dont les chaînons hétérocycliques sont choisis parmi les radicaux -O-, -N(R₆)- et -S-, R₆ représentant un atome d'hydrogène, un alkyle linéaire ou ramifié ayant de 1 à 6 atomes de carbone ou la liaison vers le cycle phényle du radical X ;
ou encore X représente un radical -(CH₂)_{q}- dans lequel q représente un entier compris entre 0 et 6 ;
et enfin Y représente l'un des radicaux 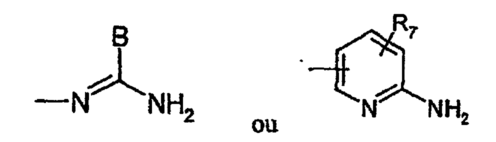 dans lesquels :
B représente un radical alkyle linéaire ou ramifié ayant de 1 à 6 atomes de carbone, aryle carbocyclique ou hétérocyclique à 5 ou 6 chaînons contenant de 1 à 4 hétéroatomes choisis parmi O, S, N et notamment les radicaux thiophène, furanne, pyrrole ou thiazole, le radical aryle étant éventuellement substitué par un ou plusieurs groupes choisis parmi les radicaux allyle, alkényle ou alkoxy linéaires ou ramifiés ayant de 1 à 6 atomes de carbone,
ou B représente NR₈R₉, dans lequel R₈ et R₉ représentent, indépendamment, un atome d'hydrogène ou un radical alkyle linéaire ou ramifié ayant de 1 à 6 atomes de carbone,
ou l'un de R₈ et R₉ représente un radical nitro tandis que l'autre représente un atome d'hydrogène ou un radical alkyle linéaire ou ramifié ayant de 1 à 6 atomes de carbone,
ou encore R₈ et R₉ pris ensemble forment avec l'atome d'azote un hétérocycle non aromatique de cinq à six chaînons, les éléments de la chaîne étant choisis parmi un groupe composé de -CH₂-, -NH-, -O- ou -S-,
ou encore B représente un radical SR₁₀ dans lequel R₁₀ représente un atome d'hydrogène ou un radical allyle linéaire ou ramifié ayant de 1 à 6 atomes de carbone,
et R₇ représente un atome d'hydrogène ou un radical alkyle linéaire ou ramifié de 1 à 6 atomes de carbone ;
ou un sel d'un produit de formule générale **(I)**.

2. Produit selon la revendication 1, **caractérisé en ce que** X représente un radical 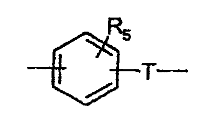 dans lequel le groupe T, qui est attaché au groupe Y, représente un radical -(CH₂)ᵢ- dans lequel i représente un entier compris entre 0 et 6, et R₅ représente un radical pyrrole, imidazole, pyrazole, triazole, thiazolidine, pyrrolidine, pipéridine, pipérazine, N-alkylpipérazine, thioniorpholine, morpholine ou azétidine.

3. Produit selon la revendication 1, **caractérisé en ce qu'**il s'agit d'un des composés suivants :
- N-{4-[[(2-thiényl)(imino)méthyl]amino]phényl}-1,2-dithiolane-3-pentanamide ;
- N-{2-{4[[(2-thiényl)(imino)méthyl]amino]phényl}éthyl}-1,2-dithiolane-3-pentanamide ;
- N-{2-{4[[(2-thiényl)(imino)méthyl]amino)phényl}éthyl}-1,2-dithiolane-3-acétamide ;
- N-[4-(6-amino-4-méthyl-2--pyridinyl)butyl]-1,2-dithiolane-3-acétamide ;
- N-{4-{[amino{2-thiényl)méthylidène]amin}phényl)-2-(1,2-dithiolan-3-yl)acétamide ;
- N-(4-{[amino(2-thiényl)méthylidène]amino}benzyl)-5-(1,2-dithiolan-3-yl)pentamide;
- N-(5-{[amino(2-thiényl)méthylidène]amino}-2-méthoxyphényl)-5-(1,2-dithiolan-3-yl)pentanamide ;
- N-[5-{(amino(2-thiényl)méthylidène]amino}2-(diméthylamino)benzyl]-5-(1,2-dithiolan-3-yl)pentanamide ;
- N-[5-{[amino(2-thiényl)méthylidène]amino}-2-(1H-pyrrol-1-yl)benzyl]-5-(1,2-dithiolan-3-yl)pentanamide ;
ou d'un sel de ces derniers.

4. Procédé de préparation d'une amidine de formule générale **(I)** selon la revendication 1, caractërisé en ce que l'on fait réagir l'intermédiaire de formule générale **(II)**. 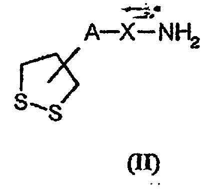 dans laquelle :
A représente l'un des radicaux -(CH₂)ₘ-CONH-(CH₂)ₙ-, (CH₂)ₘ-NR₁-(CH₂)ₙ-, -(CH₂)ₘ-CONH-(CH₂)ₚ-NR₁-(CH₂)ₙ- ou -(CH₂)ₘ-,
m et n étant des entiers compris entre 0 et 6,
p étant un entier compris entre 2 et 6,
et R₁ représentant un atome d'hydrogène ou un radical alkyle linéaire ou ramifié de l à 6 atomes de carbone ;
et X représente un radical phénylène ou -(CH₂)_{q}- dans lequel q représente un entier compris entre 0 et 6,
avec l'intermédiaire de formule générale **(I.i)** 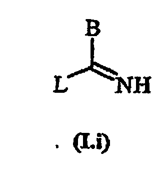 dans laquelle :
B représente un radical alkyle linéaire ou ramifié ayant de 1 à 6 atomes de carbone, aryle carbocyclique ou hétérocyclique à 5 ou 6 chaînons contenant de 1 à 4 hétéroatomes choisis parmi O, S, N et notamment les radicaux thiophène, furanne, pyrrole ou thiazole, le radical aryle étant éventuellement substitué par un ou plusieurs groupes choisis parmi les radicaux alkyle, aïkényle ou alkoxy linéaires ou ramifiés ayant de 1 à 6 atomes de carbone,
et L représente un groupe partant, par exemple un radical alkoxy, alkylthio, acide sulfonique, halogénure, alcool arylique ou tosyle.

5. Procédé de préparation d'un produit de formule générale **(I)** selon la revendication 1 dans laquelle B est une amine, **caractérisé en ce que** l'on fait réagir l'intermédiaire de formule générale **(II)** 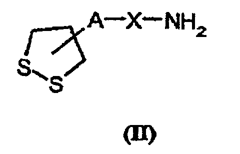 dans laquelle :
A représente l'un des radicaux -(CH₂)ₘ-CONH-(CH₂)ₙ-, (CH₂)ₘ-NR₁-(CH₂)ₙ-, (CH₂)ₘ-CONH-(CH₂)ₚ-NR₁-(CH₂)ₙ- ou -(CH₂)ₘ-,
m et n étant des entiers compris entre 0 et 6,
p étant un entier compris entre 2 et 6,
et R₁ représentant un atome d'hydrogène ou un radical alkyle linéaire ou ramifié de 1 à 6 atomes de carbone ;
et X représente un radical phénylène ou -(CH₂)_{q} dans lequel q représente un entier compris entre 0 et 6,
a) avec l'intermédiaire de formule générale **(I.ii)** 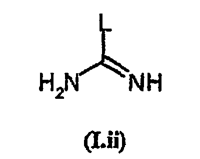 dans laquelle L représente un groupe partant, par exemple un radical alkoxy, alkylthio, acide sulfonique, halogénure, alcool arylique ou tosyle,
b) ou avec l'intermédiaire de formule générale **(I.iii)** 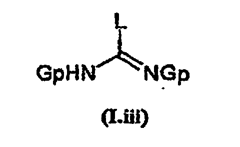 dans laquelle L représente un groupe partant, par exemple un radical alkoxy, alkylthio, acide sulfonique, halogénure, alcool arylique ou tosyle, et Gp un groupe protecteur de type carbamate, par exemple le groupe t-butoxycarbonyle,
cette réaction étant suivie, dans le cas où l'on opte pour la réaction avec le composé de formule générale **(I.iii)**, par une hydrolyse en présence d'un acide fort, par exemple l'acide trifluoroacétique,
c) ou avec le dérivé de formule **(I.iv)** (N-méthyl-N'-nitro-N-nitrosoguanidine) 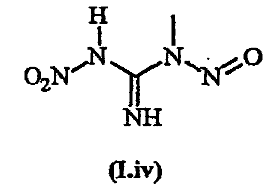
d) ou enfin avec le dérivé de formule **(I.v)** dans laquelle Gp représente un groupe protecteur
S=· =N-Gp .**(I.v)**

6. Procédé de préparation d'un composé de formule générale **(I)** selon la revendication 1, dans laquelle A représente un radical (CH₂)ₘ-CONR₃-(CH₂)ₙ- dans lequel R₃ représente un atome d'hydrogène ou un radical alkyle linéaire ou ramifié de 1 à 6 atomes de carbone, **caractérisé en ce que** l'on fait réagir l'intermédiaire de formule générale **(VII)** a représentée ci-dessous
HN(R₃)-A'-X-Y **(VII)a**
R₃ étant tel que défini ci-dessus, X et Y étant tels que définis dans la revendication 1 et A' représentant le radical -(CH₂)ₙ,-, n étant un entier de 0 à 6, avec le composé de formule générale **(I.vi)** 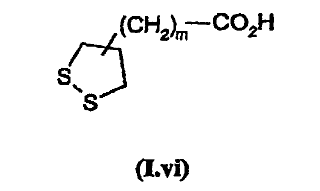 dans lequel m est un entier de 0 à 6.

7. Procédé de préparation d'un composé de formule générale **(I)** selon la revendication 1, dans laquelle A représente un radical -(CH₂)ₘ-NR₃-CO-NR₄-(CH₂)ₙ tel que défini précédemment, **caractérisé en ce que** l'on fait réagir l'intermédiaire, de formule générale **(VII)b** représentée ci-dessous
HN(R₄)-A'-X-Y **(VII)b**
R₄ étant tel que défini ci-dessus, X et Y étant tels que définis dans la revendication 1 et A' représentant le radical -(CH₂)ₙ-, n étant un entier de 0 à 6, avec le composé de formule générale **(I.vi)** 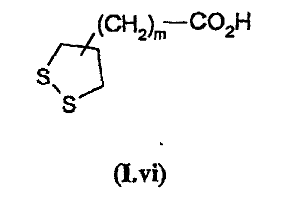 m étant un entier de 0 à 6, et avec du diphénylphosphorylazide en présence d'une base comme par exemple la triéthylamine.

8. A titre de médicament, un produit de formule générale **(I)** selon l'une des revendications 1 à 3, ou un sel pharmaceutiquement acceptable de ce dernier.

9. Composition pharmaceutique contenant à titre de principe actif au moins un produit selon l'une des revendications 1 à 3, ou un sel pharmaceutiquement acceptable dudit produit.

10. Utilisation d'un produit de formule générale **(I)** selon l'une des revendications 1 à 3, ou d'un sel pharmaceutiquement acceptable de ce produit, pour fabriquer un médicament ayant à la fois une activité d'inhibition de la NO synthase et de régénération d'anti-oxydants.

11. Utilisation d'un produit de formule générale **(I)** selon l'une des revendications 1 à 3, ou d'un sel pharmaceutiquement acceptable de ce produit, pour fabriquer un médicament destiné à traiter des pathologies dans lesquelles le monoxyde d'azote et le statut rédox des groupements thiols sont impliqués, pathologies telles que les troubles du système nerveux central ou périphérique particulièrement bien représentés par la maladie de Parkinson, les troubles cérébrovasculaires, les maladies prolifératives et inflammatoires, les vomissements, le choc septique, les pathologies résultant des irradiations radioactives, des radiations solaires ou des transplantations d'organes, les maladies autoimmunes et autosomales, les cancers et toutes les pathologies **caractérisées par** une production ou un dysfonctionnement impliquant le monoxyde d'azote et le statut rédox des groupements thiols.

12. Utilisation d'un produit de formule générale **(I)** selon l'une des revendications 1 à 3, ou d'un sel pharmaceutiquement acceptable de ce produit, pour fabriquer un médicament destiné à traiter des troubles cérébrovasculaires tels que la migraine, les infarctus cérébraux d'origine ischémique ou hémorragique, les ischémies et les thromboses.

13. Utilisation d'un produit de formule générale **(I)** selon l'une des revendications 1 à 3, ou d'un sel pharmaceutiquement acceptable de ce produit, pour fabriquer un médicament destiné à traiter les troubles du système nerveux central ou périphériques tels que les maladies neurodégénératives, la douleur, les traumatismes cérébraux ou de la moelle épinière, l'addiction aux drogues opiacées, à l'alcool et aux substances induisant une accoutumance, les troubles de l'érection et de la reproduction, les désordres cognitifs, les encéphalopathies, la dépression, l'anxiété, la schizophrénie, l'épilepsie, les troubles du sommeil et les troubles alimentaires.

## Claims

1. Product of general formula **(I)**, **characterized in that** it comprises the products of sub-formulae **(I)a** and **(I)b** 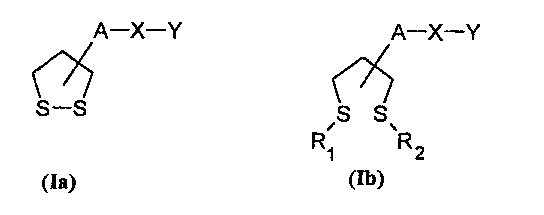 in which
R₁ and R₂ represent independently a hydrogen atom or a linear or branched alkyl radical with 1 to 6 carbon atoms;
A represents one of the -(CH₂)ₘ-NR₃-CO(CH₂)ₙ-, -(CH₂)ₘ-CO-NR₃-(CH₂)ₙ-, -(CH₂)ₘ-NR₃-(CH₂)ₙ-, -(CH₂)ₘ-CO-NR₃-(CH₂)ₚ-NR₄-(CH₂)ₙ-, -(CH₂)ₘ-NR₃-CO-NR₄-(CH₂)ₙ- or -(CH₂)ₘ- radicals,
m and n being integers from 0 to 6,
p being an integer from 2 to 6,
and R₃ and R₄ representing independently a hydrogen atom or a linear or branched alkyl radical with 1 to 6 carbon atoms;
X represents a 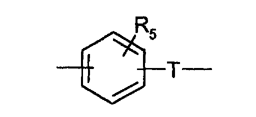 radical in which the T group, which is attached to the Y group, represents a -(CH₂)ᵢ- radical in which i represents an integer between 0 and 6, and R₅ represents a hydrogen atom, a linear or branched alkyl radical with 1 to 6 carbon atoms or a -(CH₂)ₘ-Q radical in which Q represents a halogen atom or a hydroxy, cyano, amino, alkoxy, alkylthio, alkylamino or dialkylamino radical, or also R₅ represents a heterocycle with 5 to 6 members of which the heterocyclic members are chosen from the -O-, -N(R₆)- and -S- radicals, R₆ representing a hydrogen atom, a linear or branched alkyl with 1 to 6 carbon atoms or the bond to the phenyl ring of the X radical;
or also X represents a -(CH₂)_{q}- radical in which q represents an integer between 0 and 6;
and finally Y represents one of the 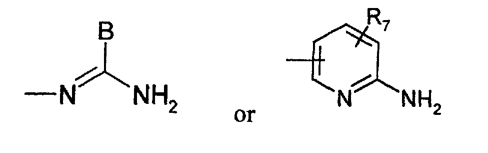 radicals in which:
B represents a linear or branched alkyl radical with 1 to 6 carbon atoms, carbocyclic or heterocyclic aryl with 5 or 6 members containing 1 to 4 heteroatoms chosen from O, S, N and in particular the thiophene, furane, pyrrole or thiazole radicals, the aryl radical being optionally substituted by one or more groups chosen from the linear or branched alkyl, alkenyl or alkoxy radicals with 1 to 6 carbon atoms,
or B represents NR₈R₉, in which R₈ and R₉ represent, independently, a hydrogen atom or a linear or branched alkyl radical with 1 to 6 carbon atoms, or one of R₈ and R₉ represents a nitro radical while the other represents a hydrogen atom or a linear or branched alkyl radical with 1 to 6 carbon atoms, or also R₈ and R₉ together form with the nitrogen atom a non-aromatic heterocycle with five to six members, the elements of the chain being chosen from a group composed of -CH₂-, -NH-, -O- or -S-,
or also B represents an SR₁₀ radical in which R₁₀ represents a hydrogen atom or a linear or branched alkyl radical with 1 to 6 carbon atoms,
and R₇ represents a hydrogen atom or a linear or branched alkyl radical with 1 to 6 carbon atoms;
or a salt of a product of general formula **(I)**.

2. Product according to claim 1, **characterized in that** X represents a 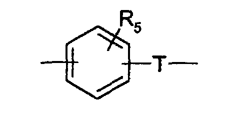 radical in which the T group, which is attached to the Y group, represents a -(CH₂)ᵢ- radical in which i represents an integer between 0 and 6, and R₅ represents a pyrrole, imidazole, pyrazole, triazole, thiazolidine, pyrrolidine, piperidine, piperazine, N-alkylpiperazine, thiomorpholine, morpholine or azetidine radical.

3. Product according to claim 1, **characterized in that** it is one of the following compounds:
- N-{4-[[(2-thienyl)(imino)methyl]amino]phenyl}-1,2-dithiolane-3-pentanamide;
- N-{2-{4[[(2-thienyl)(imino)methyl]amino]phenyl}ethyl}-1,2-dithiolane-3-pentanamide;
- N-{2-{4[[(2-thienyl)(imino)methyl]amino]phenyl}ethyl}-1,2-dithiolane-3-acetamide;
- N-[4-(6-amino-4-methyl-2-pyridinyl)butyl]-1,2-dithiolane-3-pentanamide;
- N-[4-(6-amino-4-methyl-2-pyridinyl)butyl]-1,2-dithiolane-3-acetamide;
- N-(4-{[amino(2-thienyl)methylidene]amino}phenyl)-2-(1,2-dithiolan-3-yl)acetamide;
- N-(4-{[amino(2-thienyl)methylidene]amino}benzyl)-5-(1,2-dithiolan-3-yl)pentanamide;
- N-(5-{[amino(2-thienyl)methylidene]amino)-2-methoxyphenyl)-5-(1,2-dithiolan-3-yl)pentanamide;
- N-[5-{[amino(2-thienyl)methylidene]amino}-2-(dimethylamino)benzyl]-5-(1,2-dithiolan-3-yl)pentanamide;
- N-[5-{[amino(2-thienyl)methylidene]amino}-2-(1H-pyrrol-1-yl)benzyl]-5-(1,2-dithiolan-3-yl)pentanamide;
or a salt of the latter.

4. Process for the preparation of an amidine of general formula **(I)** according to claim 1, **characterized in that** the intermediate of general formula **(II)** 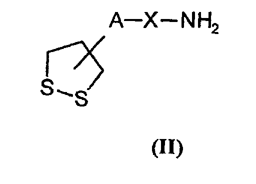 in which:
A represents one of the -(CH₂)ₘ-CONH-(CH₂)ₙ-, -(CH₂)ₘ-NR₁-(CH₂)ₙ-, (CH₂)ₘ-CONH-(CH₂)ₚ-NR₁-(CH₂)ₙ or -(CH₂)ₘ- radicals,
m and n being integers between 0 and 6,
p being an integer between 2 and 6,
and R₁ representing a hydrogen atom or a linear or branched alkyl radical with 1 to 6 carbon atoms;
and X represents a phenylene or -(CH₂)_{q}- radical in which q represents an integer between 0 and 6,
is reacted with the intermediate of general formula **(I.i)** 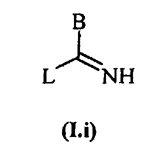 in which:
B represents a linear or branched alkyl radical with 1 to 6 carbon atoms, carbocyclic or heterocyclic aryl with 5 or 6 members containing 1 to 4 heteroatoms chosen from O, S, N and in particular the thiophene, furane, pyrrole or thiazole radicals, the aryl radical being optionally substituted by one or more groups chosen from the linear or branched alkyl, alkenyl or alkoxy radicals with 1 to 6 carbon atoms,
and L represents a parting group, for example an alkoxy, alkylthio, sulphonic acid, halide, aryl alcohol or tosyl radical.

5. Process for the preparation of a product of general formula **(I)** according to claim 1 in which B is an amine, **characterized in that** the intermediate of general formula **(II)** 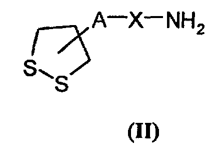 in which:
A represents one of the -(CH₂)ₘ-CONH-(CH₂)ₙ-, -(CH₂)ₘ-NR₁-(CH₂)ₙ-, -(CH₂)ₘ-CONH-(CH₂)ₚ-NR₁-(CH₂)ₙ- or -(CH₂)ₘ- radicals,
m and n being integers between 0 and 6,
p being an integer between 2 and 6,
and R₁ representing a hydrogen atom or a linear or branched alkyl radical with 1 to 6 carbon atoms;
and X represents a phenylene or -(CH₂)_{q}- radical in which q represents an integer between 0 and 6,
is reacted
a) with the intermediate of general formula **(I.ii)** 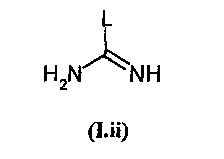 in which L represents a parting group, for example an alkoxy, alkylthio, sulphonic acid, halide, aryl alcohol or tosyl radical,
b) or with the intermediate of general formula **(I.iii)** 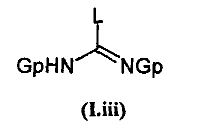 in which L represents a parting group, for example an alkoxy, alkylthio, sulphonic acid, halide, aryl alcohol or tosyl radical, and Gp a protective group of carbamate type, for example the t-butoxycarbonyl group,
this reaction being followed, in the case where reaction with the compound of general formula **(I.iii)** is chosen, by hydrolysis in the presence of a strong acid, for example trifluoroacetic acid,
c) or with the derivative of formula **(I.iv**) (N-methyl-N'-nitro-N-nitrosoguanidine) 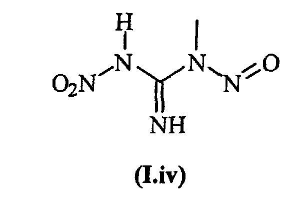
d) or finally with the derivative of formula **(I.v)** in which Gp represents a protective group
S=· =N-Gp. **(I.v).**

6. Process for the preparation of a compound of general formula **(I)** according to claim 1, in which A represents a -(CH₂)ₘ-CONR₃-(CH₂)ₙ- radical in which R₃ represents a hydrogen atom or a linear or branched alkyl radical with 1 to 6 carbon atoms, **characterized in that** the intermediate of general formula **(VII)a** represented below
HN(R₃)-A'-X-Y **(VII)a**
R₃ being as defined above, X and Y being as defined in claim 1 and A' representing the -(CH₂)ₙ- radical, n being an integer from 0 to 6, is reacted with the compound of general formula **(I.vi)** 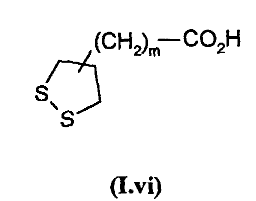 in which m is an integer from 0 to 6.

7. Process for the preparation of a compound of general formula **(I)** according to claim 1, in which A represents a -(CH₂)ₘ-NR₃-CO-NR₄-(CH₂)ₙ radical as defined previously, **characterized in that** the intermediate of general formula **(VII)b** represented below
HN(R₄)-A'-X-Y **(VII)b**
R₄ being as defined above, X and Y being as defined in claim 1 and A' representing the -(CH₂)ₙ- radical, n being an integer from 0 to 6, is reacted with the compound of general formula **(I.vi)** 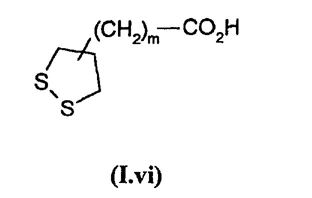 m being an integer from 0 to 6, and with diphenylphosphorylazide in the presence of a base such as for example triethylamine.

8. As a medicament, a product of general formula **(I)** according to one of claims 1 to 3, or a pharmaceutically acceptable salt of the latter.

9. Pharmaceutical composition containing as active ingredient at least one product according to one of claims 1 to 3, or a pharmaceutically acceptable salt of said product.

10. Use of a product of general formula **(I)** according to one of claims 1 to 3, or of a pharmaceutically acceptable salt of this product, for producing a medicament having both an inhibition activity on NO synthase and a regeneration activity on anti-oxidants.

11. Use of a product of general formula **(I)** according to one of claims 1 to 3, or of a pharmaceutically acceptable salt of this product, for producing a medicament intended to treat pathologies in which nitrogen monoxide and the redox status of thiol groups are involved, pathologies such as disorders of the central or peripheral nervous system particularly well represented by Parkinson's disease, cerebrovascular disorders, proliferative and inflammatory diseases, vomiting, septic shock, pathologies resulting from radioactive irradiation, solar radiation or organ transplants, autoimmune and autosomal diseases, cancers and all the pathologies **characterized by** a production or a dysfunction involving nitrogen monoxide and the redox status of thiol groups.

12. Use of a product of general formula **(I)** according to one of claims 1 to 3, or of a pharmaceutically acceptable salt of this product, for producing a medicament intended to treat cerebrovascular disorders such as migraine, cerebral infarctions of ischemic or hemorragic origin, ischemias and thromboses.

13. Use of a product of general formula **(I)** according to one of claims 1 to 3, or of a pharmaceutically acceptable salt of this product, for producing a medicament intended to treat disorders of the central or peripheral nervous system such as neurodegenerative diseases, pain, cerebral or spinal cord traumas, addiction to opiates, alcohol and addictive substances, erective and reproductive disorders, cognitive disorders, encephalopathies, depression, anxiety, schizophrenia, epilepsy, sleeping disorders and eating disorders.

## Patentansprüche

1. Produkt der allgemeinen Formel **(I)**, **dadurch gekennzeichnet, daß** sie die Produkte der Unterformeln **(I)a** und **(I)b** umfaßt 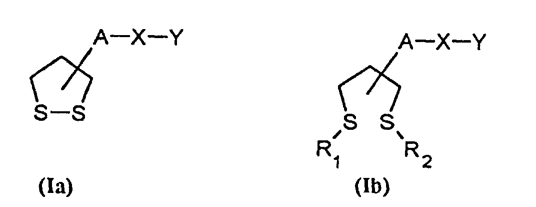 in denen
R₁ und R₂ unabhängig voneinander ein Wasserstoffatom oder einen verzweigten oder unverzweigten Alkylrest mit 1 bis 6 Kohlenstoffatomen darstellen;
A einen der Reste -(CH₂)ₘNR₃-(CO(CH₂)ₙ-,
-(CH₂)ₘ-CO-NR₃-(CH₂)ₙ-, -(CH₂)ₘ-NR₃-(CH₂)ₙ-,
-(CH₂)ₘ-CO-NR₃-(CH₂)ₚ-NR₄-(CH₂)ₙ-, -(CH₂)ₘ-NR₃-CO-NR₄-(CH₂)ₙ- oder -(CH₂)ₘ- darstellt,
wobei m und n ganze Zahlen von 0 bis 6 sind,
p eine ganze Zahl von 2 bis 6 ist,
und R₃ und R₄ unabhängig voneinander ein Wasserstoffatom oder einen verzweigten oder unverzweigten Alkylrest mit 1 bis 6 Kohlenstoffatomen darstellen;
X einen Rest 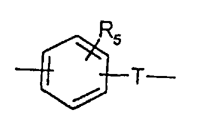 darstellt, in dem die Gruppe T, die an die Gruppe Y gebunden ist, einen Rest -(CH₂)ᵢ- darstellt, in dem i eine ganze Zahl zwischen 0 und 6 darstellt, und R₅ ein Wasserstoffatom, einen verzweigten oder unverzweigten Alkylrest mit 1 bis 6 Kohlenstoffatomen oder einen Rest -(CH₂)ₘ-Q darstellt, in dem Q ein Halogenatom oder einen der Reste Hydroxy, Cyano, Amino, Alkoxy, Alkylthio, Alkylamino oder Dialkylamino darstellt, oder R₅ einen Heterocyclus von 5 bis 6 Kettengliedern darstellt, deren heterocyclische Kettenglieder aus den Resten -O-, -N(R₆)- und -S- ausgewählt sind, wobei R₆ ein Wasserstoffatom, ein verzweigtes oder unverzweigtes Alkyl mit 1 bis 6 Kohlenstoffatomen oder die Bindung zum Phenylcyclus des Rests X darstellt;
oder X einen Rest -(CH₂)_{q}- darstellt, in dem q eine ganze Zahl zwischen 0 und 6 darstellt, und Y einen der Reste 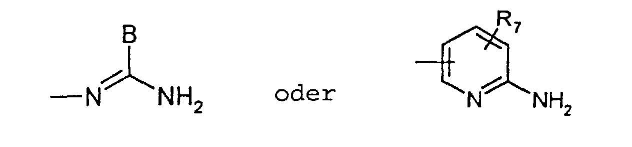 darstellt, in denen:
B einen verzweigten oder unverzweigten Alkylrest mit 1 bis 6 Kohlenstoffatomen, carbocyclisches oder heterocyclisches Aryl mit 5 bis 6 Kettengliedern mit 1 bis 4 Heteroatomen, die aus O, S, N ausgewählt sind, und insbesondere die Reste Thiophen, Furan, Pyrrol oder Thiazol darstellt, wobei der Arylrest ggf. durch eine oder mehrere Gruppen substituiert ist, die aus den Resten Alkyl, Alkenyl oder verzweigtes oder unverzweigtes Alkoxy mit 1 bis 6 Kohlenstoffatomen ausgewählt sind,
oder B NR₈R₉ darstellt, in dem R₈ und R₉ unabhängig voneinander ein Wasserstoffatom oder einen verzweigten oder unverzweigten Alkylrest mit 1 bis 6 Kohlenstoffatomen darstellen oder einer der Reste R₈ und R₉ einen Nitrorest darstellt, während der andere ein Wasserstoffatom oder einen verzweigten oder unverzweigten Alkylrest mit 1 bis 6 Kohlenstoffatomen darstellt oder R₈ und R₉ zusammen mit dem Stickstoffatom einen nichtaromatischen Heterocyclus mit fünf bis sechs Kettengliedern bilden, wobei die Elemente der Kette aus einer Gruppe ausgewählt sind, die aus -CH₂-, -NH-, -O- oder -S- besteht,
oder B einen Rest SR₁₀ darstellt, in dem R₁₀ ein Wasserstoffatom oder einen verzweigten oder unverzweigten Alkylrest mit 1 bis 6 Kohlenstoffatomen darstellt und R₇ ein Wasserstoffatom oder einen verzweigten oder unverzweigten Alkylrest mit 1 bis 6 Kohlenstoffatomen darstellt;
oder ein Salz eines Produkts der allgemeinen Formel **(I)**.

2. Produkt nach Anspruch 1, **dadurch gekennzeichnet, daß** X einen Rest 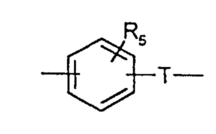 darstellt,
in dem die Gruppe T, die an die Gruppe Y gebunden ist, einen Rest -(CH₂)ᵢ darstellt, in dem i eine ganze Zahl zwischen 0 und 6 darstellt, und R₅ einen der Reste Pyrrol, Imidazol, Pyrazol, Triazol, Thiazolidin, Pyrrolidin, Piperidin, Piperazin, N-Alkylpiperazin, Thiomorpholin, Morpholin oder Azetidin darstellt.

3. Produkt nach Anspruch 1, **dadurch gekennzeichnet, daß** es sich um eine der folgenden Verbindungen:
- N-{4-[[(2-Thienyl)(imino)methyl]amino]phenyl}-1,2-dithiolan-3-pentanamid;
- N-{2-{4[[(2-Thienyl)(imino)methyl]amino]phenyl}ethyl}-1,2-dithiolan-3-pentanamid;
- N-{2-{4[[(2-Thienyl)(imino)methyl]amino]phenyl}ethyl}-1,2-dithiolan-3-acetamid;
- N-[4-(6-Amino-4-methyl-2-pyridinyl)butyl]-1,2-dithiolan-3-pentanamid;
- N-[4-(6-Amino-4-methyl-2-pyridinyl)butyl]-1,2-dithiolan-3-acetamid;
- N-(4-{[Amino(2-thienyl)methyliden]amino}phenyl)-2-(1,2-dithiolan-3-yl)acetamid;
- N-(4-{[Amino(2-thienyl)methyliden]amino}benzyl)-5-(1,2-dithiolan-3-yl)pentanamid;
- N-(5-{[Amino(2-thienyl)methyliden]amino}-2-(methoxyphenyl)-5-(1,2-dithiolan-3-yl)pentanamid;
- N-[5-{[Amino(2-thienyl)methyliden]amino}-2-(dimethylamino)benzyl]-5-(1,2-dithiolan-3-yl)pentanamid;
- N-[5-{[Amino(2-thienyl)methyliden]amino}-2-(1H-pyrrol-1-yl)benzyl]-5-(1,2-dithiolan-3-yl)pentanamid;
oder um ein Salz von diesen handelt.

4. Verfahren zur Herstellung eines Amidins der allgemeinen Formel (I) nach Anspruch 1, **dadurch gekennzeichnet, daß** man das Zwischenprodukt der allgemeinen Formel **(II)** 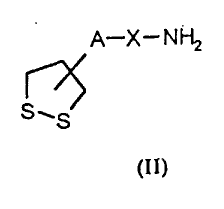 in der
A einen der Reste - (CH₂)ₘ-CONH-(CH₂)ₙ-, - (CH₂)ₘ-NR₁-(CH₂)ₙ-, -(CH₂)ₘ-CONH-(CH₂)ₚ-NR₁-(CH₂)ₙ- oder -(CH₂)ₘ- darstellt, wobei m und n ganze Zahlen zwischen 0 und 6 sind,
p eine ganze Zahl zwischen 2 und 6 ist
und R₁ ein Wasserstoffatom oder einen verzweigten oder unverzweigten Alkylrest mit 1 bis 6 Kohlenstoffatomen darstellt;
und X einen der Reste Phenylen oder -(CH₂)_{q}- darstellt, in dem q eine ganze Zahl zwischen 0 und 6 darstellt,
mit dem Zwischenprodukt der allgemeinen Formel **(I.i)** reagieren läßt, 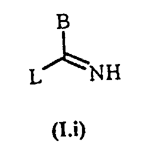 in der
B einen verzweigten oder unverzweigten Alkylrest mit 1 bis 6 Kohlenstoffatomen, carbocyclisches oder heterocyclisches Aryl mit 5 bis 6 Kettengliedern mit 1 bis 4 Heteroatomen, die aus O, S, N ausgewählt sind, und insbesondere die Reste Thiophen, Furan, Pyrrol oder Thiazol darstellt, wobei der Arylrest ggf. durch eine oder mehrere Gruppen substituiert ist, die aus den verzweigten oder unverzweigten Resten Alkyl, Alkenyl oder Alkoxy mit 1 bis 6 Kohlenstoffatomen ausgewählt sind,
und L eine Abgangsgruppe darstellt, beispielsweise einen der Reste Alkoxy, Alkylthio, Sulfonsäure, Halogenid, Arylalkohol oder Tosyl.

5. Verfahren zur Herstellung eines Produkts der allgemeinen Formel **(I)** nach Anspruch 1, in der B ein Amin ist, **dadurch gekennzeichnet, daß** man das Zwischenprodukt der allgemeinen Formel **(II)** 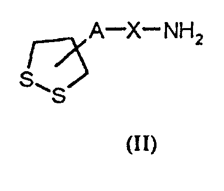 in der
A einen der Reste - (CH₂)ₘ-CONH-(CH₂)ₙ- , -(CH₂)ₘNR₁-(CH₂)ₙ-, -(CH₂)ₘ-CONH-(CH₂)ₚ-NR₁-(CH₂)ₙ- oder -(CH₂)ₘ- darstellt, wobei m und n ganze Zahlen zwischen 0 und 6 sind,
p eine ganze Zahl zwischen 2 und 6 ist
und R₁ ein Wasserstoffatom oder einen verzweigten oder unverzweigten Alkylrest mit 1 bis 6 Kohlenstoffatomen darstellt;
und X einen der Reste Phenylen oder -(CH₂)_{q}- darstellt, in dem q eine ganze Zahl zwischen 0 und 6 darstellt,
a) mit dem Zwischenprodukt der allgemeinen Formel **(I.ii)** reagieren läßt, 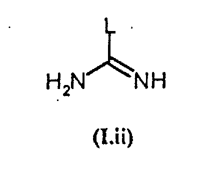 in der L eine Abgangsgruppe darstellt, beispielsweise einen der Reste Alkoxy, Alkylthio, Sulfonsäure, Halogenid, Arylalkohol oder Tosyl,
b) oder mit dem Zwischenprodukt der allgemeinen Formel **(I.iii)** reagieren läßt, 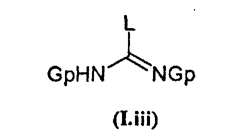 in der L eine Abgangsgruppe, beispielsweise einen der Reste Alkoxy, Alkylthio, Sulfonsäure, Halogenid, Arylalkohol oder Tosyl, und Gp eine Schutzgruppe vom Typ Carbamat, beispielsweise die t-Butoxycarbonyl-Gruppe, darstellt,
wobei auf diese Reaktion, wenn man die Reaktion mit der Verbindung der allgemeinen Formel **(I,iii)** wählt, eine Hydrolyse in Gegenwart einer starken Säure, beispielsweise Trifluoressigsäure, folgt,
c) oder mit dem Derivat der Formel **(I.iv)** (N-Methyl-N'nitro-N-nitrosoguanidin) reagieren läßt 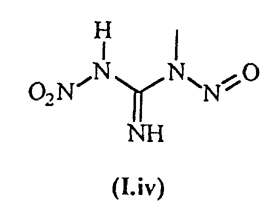
d) oder mit dem Derivat der Formel **(I.v)** reagieren läßt, in der Gp eine Schutzgruppe darstellt
S=· =N-Gp. **(I.v)**

6. Verfahren zur Herstellung einer Verbindung der allgemeinen Formel (I) nach Anspruch 1, in der A einen Rest -(CH₂)ₘ-CONR₃-(CH₂)ₙ- darstellt, in dem R₃ ein Wasserstoffatom oder einen verzweigten oder unverzweigten Alkylrest mit 1 bis 6 Kohlenstoffatomen darstellt, **dadurch gekennzeichnet, daß** man das Zwischenprodukt der unten dargestellten allgemeinen Formel **(VII)a**
HN(R₃)-A'-X-Y **(VII)a**
worin R₃ die oben definierte Bedeutung hat, X und Y die in Anspruch 1 definierte Bedeutung haben und A' den Rest -(CH₂)ₙ- darstellt, wobei n eine ganze Zahl von 0 bis 6 ist, mit der Verbindung der allgemeinen Formel **(I.vi)** 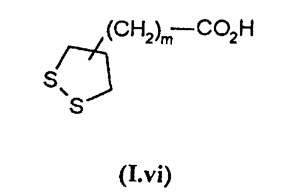 reagieren läßt,
in der m eine ganze Zahl von 0 bis 6 ist.

7. Verfahren zur Herstellung einer Verbindung der allgemeinen Formel **(I)** nach Anspruch 1, in der A einen Rest -(CH₂)ₘ-NR₃-CO-NR₄-(CH₂)ₙ- der oben definierten Art darstellt, **dadurch gekennzeichnet, daß** man das Zwischenprodukt der unten dargestellten allgemeinen Formel **(VII)b**
HN(R₄)-A'-X-Y **(VII)b**
in der R₄ die oben angegebene Bedeutung hat, X und Y die in Anspruch 1 angegebene Bedeutung haben und A' den Rest -(CH₂)ₙ- darstellt, wobei n eine ganze Zahl von 0 bis 6 ist, mit der Verbindung der allgemeinen Formel **(I.vi)** 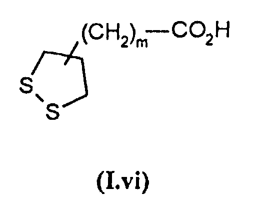 worin m eine ganze Zahl von 0 bis 6 ist, und mit Diphenylphosphorylazid in Gegenwart einer Base, wie z.B. Triethylamin, reagieren läßt.

8. Ein Produkt der allgemeinen Formel **(I)** nach einem der Ansprüche 1 bis 3 oder ein pharmazeutisch verträgliches Salz von diesem Produkt in Form eines Medikaments.

9. Pharmazeutische Zusammensetzung, die als Wirkstoff mindestens ein Produkt nach einem der Ansprüche 1 bis 3 oder ein pharmazeutisch verträgliches Salz von diesem enthält.

10. Verwendung eines Produkts der allgemeinen Formel **(I)** nach einem der Ansprüche 1 bis 3 oder eines pharmazeutisch verträglichen Salzes dieses Produkts zur Herstellung eines Medikaments, das gleichzeitig eine Wirkung der Hemmung der NO-Synthase und der Regenerierung von Antioxidantien besitzt.

11. Verwendung eines Produkts der allgemeinen Formel **(I)** nach einem der Ansprüche 1 bis 3 oder eines pharmazeutisch verträglichen Salzes dieses Produkts zur Herstellung eines Medikaments, das zur Behandlung von Erkrankungen bestimmt ist, an denen Stickstoffmonoxid und der Redoxstatus der Thiolgruppen beteiligt sind, Erkrankungen, wie z.B. Störungen des zentralen oder peripheren Nervensystems, inbesondere Parkinson, zerebrovaskuläre Störungen, proliferative und entzündliche Erkrankungen, Erbrechen, septischer Schock, Krankheiten, die sich aus radioaktiven Bestrahlungen, Sonnenbestrahlungen oder Organtransplantationen ergeben, Autoimmunkrankheiten und autosomale Krankheiten, Karzinome und alle Krankheiten, die durch eine Produktion oder Dysfunktion gekennzeichnet sind, an der Stickstoffmonoxid und der Redoxstatus der Thiolgruppen beteiligt sind.

12. Verwendung eines Produkts der allgemeinen Formel **(I)** nach einem der Ansprüche 1 bis 3 oder eines pharmazeutisch verträglichen Salzes dieses Produkts für die Herstellung eines Medikaments, das für die Behandlung von zerebrovaskulären Störungen, wie z.B. Migräne, Hirninfarkte ischämischen oder hämorrhagischen Ursprungs, Ischämien und Thrombosen, bestimmt ist.

13. Verwendung eines Produkts der allgemeinen Formel **(I)** nach einem der Ansprüche 1 bis 3 oder eines pharmazeutisch verträglichen Salzes dieses Produkts für die Herstellung eines Medikaments, das für die Behandlung von Störungen des zentralen oder peripheren Nervensystems, wie z.B. neurodegenerative Krankheiten, Schmerz, Traumatismen des Gehirns oder des Rückenmarks, Sucht gegenüber opiumhaltigen Drogen, Alkohol und Abhängigkeit erzeugenden Substanzen, Erektionsund Fortpflanzungsstörungen, kognitive Störungen, Enzephalopathien, Depression, Angstzustände, Schizophrenie, Epilepsie, Schlafstörungen und Eßstörungen, bestimmt ist.
